# EUROPEAN PATENT SPECIFICATION

(11) **EP 1 898 882 B1**
(45) Date of publication and mention of the grant of the patent: **28.10.2009**
(21) Application number: 06772583.8
(22) Date of filing: 08.06.2006
(51) Int. Cl.: A61K 9/14, A61K 31/445

(54) **NANOPARTICULATE EBASTINE FORMULATIONS**
NANOPARTIKULÄRE EBASTINFORMULIERUNGEN
FORMULATIONS D'EBASTINE NANOPARTICULAIRE

(30) Priority: 09.06.2005 US 688955 P
(43) Date of publication of application: 19.03.2008
(73) Proprietor: Elan Pharma International Limited, Athlone Westmeath (IE)
(72) Inventor: LIVERSIDGE, Gary, G., West Chester, PA 19380 (US); JENKINS, Scott, Downingtown, PA 19335 (US)
(74) Representative: Wichmann, Hendrik
(86) International application number: PCT/US2006/022330
(87) International publication number: WO 2006/135689

(56) References cited:
- EP-A- 1 552 851
- US-A- 5 460 829
- US-A1- 2005 042 177

## Description

### FIELD OF THE INVENTION

The present invention relates to H1-histamine receptor antagonist compounds and compositions useful in the treatment or prevention of disease or disorders such as seasonal and perennial allergic rhinitis, idiopathic chronic urticaria and related conditions or symptoms. More specifically, the invention relates to nanoparticulate H1-histamine receptor antagonist compositions, such as nanoparticulate ebastine compositions with an average effective particle size of less than about 2000 nm. The invention also relates to methods of making and using such nanoparticulate compositions.

### BACKGROUND OF THE INVENTION

Antihistamines have long been used to treat the symptoms of seasonal and chronic allergies, (seasonal and perennial allergic rhinitis) hay fever, and hives, also known as urticaria (*see e.g*., Glenis Scadding, Clin. Drug Invest. 2005, 25(3):153-164). Conditions and diseases such these have now been recognized as debilitating medical problems that can have an enormous adverse effect on a patient's quality of life. (*See e.g*., Corren J, J. Allergy Clin. Immunol. 2000, 105(6):610-615).

In an allergic reaction, antihistamines act as reversible antagonists of histamine. Basically, antihistamines prevent histamines, released by mast cells or basophils in response to an allergen, from binding to the H1-histamine receptors. Once histamine is released and bound to its receptor, it can have wide-ranging effects on the body which may include runny nose, itching, and sneezing

There are numerous categories of antihistamines, some having more severe secondary symptoms (*e*.*g*., sedation and gastrointestinal distress) than others. "First-generation" antihistamines (such as ethanolamines and alkylamines) have been shown to cause moderate to severe side effects, including sedation, anticholinergic adverse effects, and some CNS dysfunction. "Second generation" antihistamines such as ebastine, loratadine and fexofenadine were developed to lessen some of these side effects. "Third generation" antihistamines, metabolites or derivatives of second generation drugs, were developed to be even more effective with even fewer side effects. With active research in the area, many second and third generation antihistamines have been shown to have medicinal functions other than just blocking histamine receptors.

### A. Background Regarding Ebastine

Ebastine, a second generation antihistamine, CAS no. 90729-43-4, is chemically known as 4'-tert-butyl-4-[4-(diphenylmethoxy)piperidino] butyrophenone. Ebastine has an empiric formula of C₃₂H₃₉NO_{2,} with a molecular weight of 469.67.

The chemical structure of ebastine is:

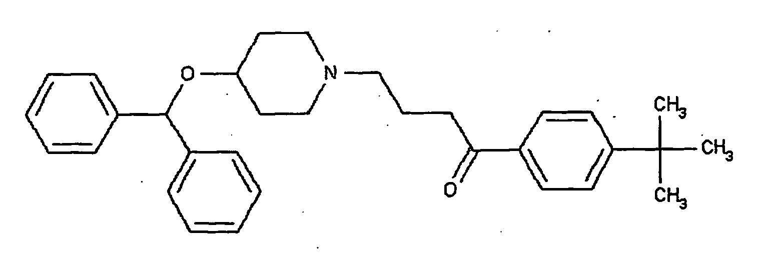

Ebastine is a long-acting and selective H1-histamine receptor antagonist. Ebastine is converted to the pharmacologically active acid metabolite, carebastine. The half-life of carebastine is between 15 and 19 hours with 66% of the medicine being excreted in the urine mainly as conjugated metabolites. Following repeated administration of 10 mg once daily, a steady state is achieved in 3 to 5 days with peak plasma levels ranging from 130 to 160 ng/mL. Ebastine is indicated for the symptomatic treatment of seasonal and perennial allergic rhinitis and idiopathic chronic urticaria.

Ebastine, generally administered once daily in strengths of 10 mg, is a non-sedating antihistamine for the treatment of symptoms associated with seasonal and perennial allergic rhinitis. Ebastine is commercially available in various countries outside of the United States under various tradenames such as Kestine™, Ebastel™, Evastel™, and No-Sedat™, marketed by such companies as Almirall Prodesfarma of Spain, in film-coated, 10 mg oral ebastine tablets.

Ebastine compounds are disclosed, for example, in United States Patent Nos. 4,550,116 for "Piperidine Derivative," 4,766,215 for "Histamine H1-histamine receptor antagonists," 5,204,249 for "Process for the Preparation of Carebastine and Similar Materials," 5,460,829 for "Pharmaceutical Compositions Based on Ebastine or Analogues Thereof," and 5,602,148 for "Liquid Compositions Based on Derivatives of 1,4 Substituted Piperidine," all of which are incorporated herein by reference in their entirety.

Ebastine is highly effective in the therapy and treatment of seasonal and perennial allergic rhinitis and related diseases. However, ebastine is not very soluble in water and as a result, it does not become readily bioavailable when given orally. Thus, it would be desirable to formulate a more soluble - and more bioavailable - form of antihistamine such as ebastine. Such a formulation would be faster acting, thereby providing relief to a subject suffering from rhinitis, urticaria and related disorders much more quickly. Such a formulation may also overcome other problems associated with conventional drug formulations. The present invention satisfies these needs.

The present invention then, relates to nanoparticulate H1 - receptor antagonist compositions, such as nanoparticulate ebastine, or a salt or derivative thereof, compositions for the treatment of seasonal and perennial allergic rhinitis, idiopathic chronic urticaria and related diseases, disorders, conditions and symptoms.

### B. Background Regarding Nanoparticulate Active Agent Compositions

Nanoparticulate active agent compositions, first described in U.S. Patent No. 5,145,684 ("the'684 patent"), are particles consisting of a poorly soluble therapeutic or diagnostic agent having adsorbed onto the surface thereof a non-crosslinked surface stabilizer. The '684 patent does not describe nanoparticulate compositions of H1-histamine receptor antagonists such as ebastine.

Methods of making nanoparticulate active agent compositions are described in, for example, U.S. Patent Nos. 5,518,187 and 5,862,999, both for "Method of Grinding Pharmaceutical Substances;" U.S. Patent No. 5,718,388, for "Continuous Method of Grinding Pharmaceutical Substances;" and U.S. Patent No. 5,510,118 for "Process of Preparing Therapeutic Compositions Containing Nanoparticles."

Nanoparticulate active agent compositions are also described, for example, in U.S. Patent Nos. 5,298,262 for "Use of Ionic Cloud Point Modifiers to Prevent Particle Aggregation During Sterilization;" 5,302,401 for "Method to Reduce Particle Size Growth During Lyophilization;" 5,318,767 for "X-Ray Contrast Compositions Useful in Medical Imaging;" 5,326,552 for "Novel Formulation For Nanoparticulate X-Ray Blood Pool Contrast Agents Using High Molecular Weight Non-ionic Surfactants;" 5,328,404 for "Method of X-Ray Imaging Using Iodinated Aromatic Propanedioates;" 5,336,507 for "Use of Charged Phospholipids to Reduce Nanoparticle Aggregation;" 5,340,564 for "Formulations Comprising Olin 10-G to Prevent Particle Aggregation and Increase Stability;" 5,346,702 for "Use of Non-lonic Cloud Point Modifiers to Minimize Nanoparticulate Aggregation During Sterilization;" 5,349,957 for "Preparation and Magnetic Properties of Very Small Magnetic-Dextran Particles;" 5,352,459 for "Use of Purified Surface Modifiers to Prevent Particle Aggregation During Sterilization;" 5,399,363 and 5,494,683, both for "Surface Modified Anticancer Nanoparticles;" 5,401,492 for "Water Insoluble Non-Magnetic Manganese Particles as Magnetic Resonance Enhancement Agents;" 5,429,824 for "Use of Tyloxapol as a Nanoparticulate Stabilizer;" 5,447,710 for "Method for Making Nanoparticulate X-Ray Blood Pool Contrast Agents Using High Molecular Weight Non-ionic Surfactants;" 5,451,393 for "X-Ray Contrast Compositions Useful in Medical Imaging;" 5,466,440 for Formulations of Oral Gastrointestinal Diagnostic X-Ray Contrast Agents in Combination with Pharmaceutically Acceptable Clays;" 5,470,583 for "Method of Preparing Nanoparticle Compositions Containing Charged Phospholipids to Reduce Aggregation;" 5,472,683 for "Nanoparticulate Diagnostic Mixed Carbamic Anhydrides as X-Ray Contrast Agents for Blood Pool and Lymphatic System Imaging;" 5,500,204 for "Nanoparticulate Diagnostic Dimers as X-Ray Contrast Agents for Blood Pool and Lymphatic System Imaging;" 5,518,738 for "Nanoparticulate NSAID Formulations;" 5,521,218 for "Nanoparticulate lododipamide Derivatives for Use as X-Ray Contrast Agents;" 5,525,328 for "Nanoparticulate Diagnostic Diatrizoxy Ester X-Ray Contrast Agents for Blood Pool and Lymphatic System Imaging;" 5,543,133 for "Process of Preparing X-Ray Contrast Compositions Containing Nanoparticles;" 5,552,160 for "Surface Modified NSAID Nanoparticles;" 5,560,931 for "Formulations of Compounds as Nanoparticulate Dispersions in Digestible Oils or Fatty Acids;" 5,565,188 for "Polyalkylene Block Copolymers as Surface Modifiers for Nanoparticles;" 5,569,448 for "Sulfated Non-ionic Block Copolymer Surfactant as Stabilizer Coatings for Nanoparticle Compositions;" 5,571,536 for "Formulations of Compounds as Nanoparticulate Dispersions in Digestible Oils or Fatty Acids;" 5,573,749 for "Nanoparticulate Diagnostic Mixed Carboxylic Anydrides as X-Ray Contrast Agents for Blood Pool and Lymphatic System Imaging;" 5,573,750 for "Diagnostic Imaging X-Ray Contrast Agents;" 5,573,783 for "Redispersible Nanoparticulate Film Matrices With Protective Overcoats;" 5,580,579 for "Site-specific Adhesion Within the GI Tract Using Nanoparticles Stabilized by High Molecular Weight, Linear Poly(ethylene Oxide) Polymers;" 5,585,108 for "Formulations of Oral Gastrointestinal Therapeutic Agents in Combination with Pharmaceutically Acceptable Clays;" 5,587,143 for "Butylene Oxide-Ethylene Oxide Block Copolymers Surfactants as Stabilizer Coatings for Nanoparticulate Compositions;" 5,591,456 for "Milled Naproxen with Hydroxypropyl Cellulose as Dispersion Stabilizer;" 5,593,657 for "Novel Barium Salt Formulations Stabilized by Non-ionic and Anionic Stabilizers;" 5,622,938 for "Sugar Based Surfactant for Nanocrystals;" 5,628,981 for "Improved Formulations of Oral Gastrointestinal Diagnostic X-Ray Contrast Agents and Oral Gastrointestinal Therapeutic Agents;" 5,643,552 for "Nanoparticulate Diagnostic Mixed Carbonic Anhydrides as X-Ray Contrast Agents for Blood Pool and Lymphatic System Imaging;" 5,718,388 for "Continuous Method of Grinding Pharmaceutical Substances;" 5,718,919 for "Nanoparticles Containing the R(-)Enantiomer of Ibuprofen;" 5,747,001 for "Aerosols Containing Beclomethasone Nanoparticle Dispersions;" 5,834,025 for "Reduction of Intravenously Administered Nanoparticulate Formulation Induced Adverse Physiological Reactions;" 6,045,829 "Nanocrystalline Formulations of Human Immunodeficiency Virus (HIV) Protease Inhibitors Using Cellulosic Surface Stabilizers;" 6,068,858 for "Methods of Making Nanocrystalline Formulations of Human Immunodeficiency Virus (HIV) Protease Inhibitors Using Cellulosic Surface Stabilizers;" 6,153,225 for "Injectable Formulations of Nanoparticulate Naproxen;" 6,165,506 for "New Solid Dose Form of Nanoparticulate Naproxen;" 6,221,400 for "Methods of Treating Mammals Using Nanocrystalline Formulations of Human Immunodeficiency Virus (HIV) Protease Inhibitors;" 6,264,922 for "Nebulized Aerosols Containing Nanoparticle Dispersions;" 6,267,989 for "Methods for Preventing Crystal Growth and Particle Aggregation in Nanoparticle Compositions;" 6,270,806 for "Use of PEG-Derivatized Lipids as Surface Stabilizers for Nanoparticulate Compositions;" 6,316,029 for "Rapidly Disintegrating Solid Oral Dosage Form," 6,375,986 for "Solid Dose Nanoparticulate Compositions Comprising a Synergistic Combination of a Polymeric Surface Stabilizer and Dioctyl Sodium Sulfosuccinate;" 6,428,814 for "Bioadhesive Nanoparticulate Compositions Having Cationic Surface Stabilizers;" 6,431,478 for "Small Scale Mill;" and 6,432,381 for "Methods for Targeting Drug Delivery to the Upper and/or Lower Gastrointestinal Tract," 6,592,903 for "Nanoparticulate Dispersions Comprising a Synergistic Combination of a Polymeric Surface Stabilizer and Dioctyl Sodium Sulfosuccinate," 6,582,285 for "Apparatus for sanitary wet milling;" 6,656,504 for "Nanoparticulate Compositions Comprising Amorphous Cyclosporine;" 6,742,734 for "System and Method for Milling Materials;" 6,745,962 for "Small Scale Mill and Method Thereof;" 6,811,767 for "Liquid droplet aerosols of nanoparticulate drugs;" 6,908,626 for "Compositions having a combination of immediate release and controlled release characteristics;" 6,969,529 for "Nanoparticulate compositions comprising copolymers of vinyl pyrrolidone and vinyl acetate as surface stabilizers;" and 6,976,647 for "System and Method for Milling Materials," all of which are specifically incorporated by reference.

In addition, U.S. Patent Application No. 20020012675 A1, published on January 31, 2002, for "Controlled Release Nanoparticulate Compositions;" U.S. Patent Publication No. 20050276974 for "Nanoparticulate Fibrate Formulations;" U.S. Patent Publication No. 20050238725 for "Nanoparticulate compositions having a peptide as a surface stabilizer;" U.S. Patent Publication No. 20050233001 for "Nanoparticulate megestrol formulations;" U.S. Patent Publication No. 20050147664 for "Compositions comprising antibodies and methods of using the same for targeting nanoparticulate active agent delivery;" U.S. Patent Publication No. 20050063913 for "Novel metaxalone compositions;" U.S. Patent Publication No. 20050042177 for "Novel compositions of sildenafil free base;" U.S. Patent Publication No. 200500316911 for "Gel stabilized nanoparticulate active agent compositions;" U.S. Patent Publication No. 20050019412 for" Novel glipizide compositions;" U.S. Patent Publication No. 20050004049 for "Novel griseofulvin compositions;" U.S. Patent Publication No. 20040258758 for "Nanoparticulate topiramate formulations;" U.S. Patent Publication No. 20040258757 for" Liquid dosage compositions of stable nanoparticulate active agents;" U.S. Patent Publication No. 20040229038 for "Nanoparticulate meloxicam formulations;" U.S. Patent Publication No. 20040208833 for "Novel fluticasone formulations;" U.S. Patent Publication No. 20040195413 for " Compositions and method for milling materials;" U.S. Patent Publication No. 20040156895 for "Solid dosage forms comprising pullulan;" U.S. Patent Publication No. U.S. Patent Publication No. U.S. Patent Publication No. 20040156872 for "Novel nimesulide compositions;" U.S. Patent Publication No. 20040141925 for "Novel triamcinolone compositions;" U.S. Patent Publication No. 20040115134 for "Novel nifedipine compositions;" U.S. Patent Publication No. 20040105889 for "Low viscosity liquid dosage forms;" U.S. Patent Publication No. 20040105778 for "Gamma irradiation of solid nanoparticulate active agents;" U.S. Patent Publication No. 20040101566 for "Novel benzoyl peroxide compositions;" U.S. Patent Publication No. 20040057905 for "Nanoparticulate beclomethasone dipropionate compositions;" U.S. Patent Publication No. 20040033267 for "Nanoparticulate compositions of angiogenesis inhibitors;" U.S. Patent Publication No. 20040033202 for "Nanoparticulate sterol formulations and novel sterol combinations;" U.S. Patent Publication No. 20040018242 for "Nanoparticulate nystatin formulations;" U.S. Patent Publication No. 20040015134 for "Drug delivery systems and methods;" U.S. Patent Publication No. 20030232796 for "Nanoparticulate polycosanol formulations & novel polycosanol combinations;" U.S. Patent Publication No. 20030215502 for "Fast dissolving dosage forms having reduced friability;" U.S. Patent Publication No. 20030185869 for "Nanoparticulate compositions having lysozyme as a surface stabilizer;" U.S. Patent Publication No. 20030181411 for "Nanoparticulate compositions of mitogen-activated protein (MAP) kinase inhibitors;" U.S. Patent Publication No. 20030137067 for "Compositions having a combination of immediate release and controlled release characteristics;" U.S. Patent Publication No. 20030108616 for "Nanoparticulate compositions comprising copolymers of vinyl pyrrolidone and vinyl acetate as surface stabilizers;" U.S. Patent Publication No. 20030095928 for "Nanoparticulate insulin;" U.S. Patent Publication No. 20030087308 for "Method for high through put screening using a small scale mill or microfluidics;" U.S. Patent Publication No. 20030023203 for "Drug delivery systems & methods;" U.S. Patent Publication No. 20020179758 for "System and method for milling materials; and U.S. Patent Publication No. 20010053664 for "Apparatus for sanitary wet milling," describe nanoparticulate active agent compositions and are specifically incorporated by reference.

Amorphous small particle compositions are described, for example, in U.S. Patent Nos. 4,783,484 for "Particulate Composition and Use Thereof as Antimicrobial Agent;" 4,826,689 for "Method for Making Uniformly Sized Particles from Water-Insoluble Organic Compounds;" 4,997,454 for "Method for Making Uniformly-Sized Particles From Insoluble Compounds;" 5,741,522 for "Ultrasmall, Non-aggregated Porous Particles of Uniform Size for Entrapping Gas Bubbles Within and Method;" and 5,776,496, for "Ultrasmall Porous Particles for Enhancing Ultrasound Back Scatter." These are also specifically incorporated herein by reference.

While the high therapeutic value of the H1-histamine receptor antagonists such as ebastine are recognized in the art, poorly soluble antagonists are limited in their bioavailability upon oral administration and can be difficult or impossible to formulate as safe and effective products for other types of administration. Thus, there is a need in the art for formulations comprising H1-histamine receptor antagonists which have improved oral bioavailability and thus improved efficacy and/or are suitable for other types of administration, such as parenteral administration. The present invention fills that need.

The present invention then, relates to nanoparticulate compositions comprising an H1-histamine receptor antagonists such as ebastine, which may be useful in the treatment and prevention of diseases and disorders, such as seasonal and perennial allergic rhinitis, idiopathic chronic urticaria and related conditions.

### SUMMARY OF THE INVENTION

The present invention relates to stable nanoparticulate compositions comprising an H₁-histamine receptor antagonist, such as ebastine, or a salt or derivative thereof, and at least one surface stabilizer. In some embodiments, the surface stabilizer may be associated with the surface of the particles, for example, the surface stabilizer may be adsorbed onto the surface of the ebastine particle. In general, the nanoparticles have an effective average particle size of less than about 2000 nm.

The compositions may include ebastine particles which are in a crystalline phase, an amorphous phase, a semi-crystalline phase, a semi-amorphous phase and mixtures thereof.

The compositions may include one or more surface stabilizers. For example, some compositions may include at least one primary and at least one secondary surface stabilizer. Exemplary surface stabilizers include, but are not limited to non-ionic surface stabilizers, ionic surface stabilizers, anionic surface stabilizers, cationic surface stabilizers, zwitterionic surface stabilizers and combinations thereof.

The invention also relates to nanoparticulate H1-histamine receptor antagonist such as ebastine or a salt or derivative thereof compositions, at least one surface stabilizer, and optionally one or more pharmaceutically acceptable excipients, carriers, and optionally one or more active agents useful for the treatment of seasonal and perennial allergic rhinitis and related diseases, or a combination thereof.

The H1-histamine receptor antagonist, such as ebastine or a salt or derivative thereof, nanoparticulate compositions of the present invention are proposed to exhibit improved pharmacokinetic profiles as compared to conventional H1-histamine receptor antagonist compositions. For example, the Cₘₐₓ and/or AUC of the nanoparticulate compositions may be greater than the Cₘₐₓ and/or AUC for conventional compositions administered at the same dosage while the Tₘₐₓ may be lower; any combination of an improved Cₘₐₓ, AUC and Tₘₐₓ profile may be exhibited by the nanoparticulate ebastine compositions as compared to conventional ebastine compositions. In further embodiments, the ebastine compositions may not produce significantly different absorption levels when administered under fed as compared to fasting conditions.

In some embodiments, the nanoparticulate ebastine compositions exhibit improved bioavailability as compared to conventional ebastine compositions. For example, upon administration to a mammal, the nanoparticulate ebastine compositions may redisperse such that the particles have an effective average particle size of less than about 2 microns.

The invention also relates to methods of making nanoparticulate compositions including an H1-histamine receptor antagonist, such as ebastine or salt or derivative thereof. In some embodiments, the methods may include contacting particles of an ebastine with at least one surface stabilizer for a time and under conditions sufficient to provide a nanoparticulate ebastine composition having an effective average particle size of less than about 2000 nm.

The invention also relates to methods of treatment using the nanoparticulate ebastine compositions. In some methods, a composition having an effective average particle size of less than about 2000 nm and including a nanoparticulate ebastine or salt or derivative thereof and at least one surface stabilizer, may be administered to a subject. In some methods, the composition may be administered orally, for example, as a tablet, in a therapeutically effective amount. By way of example, but not by way of limitation, the composition may be administered to treat diseases, disorders, symptoms or conditions such as seasonal and perennial allergic rhinitis, idiopathic chronic urticaria or a related disease, disorders, symptoms or conditions. In other methods, the subject may be suffering from such a disease, disorder, symptom or condition. Other methods of treatment using the nanoparticulate compositions of the invention are known to those of skill in the art.

Both the foregoing summary of the invention and the following detailed description of the invention are exemplary and explanatory and are intended to provide further details of the invention as claimed. Other objects, advantages, and novel features will be readily apparent to those skilled in the art from the following detailed description of the invention.

### DETAILED DESCRIPTION OF THE INVENTION

The present invention is directed to nanoparticulate antihistamine compositions comprising ebastine, or a salt or derivative thereof. The compositions comprise a nanoparticulate ebastine, or a salt or derivative thereof, and at least one surface stabilizer. The surface stabilizer may be adsorbed or associated with the surface of the drug. Generally, the ebastine particles, or a salt or derivative thereof, have an effective average particle size of less than about 2000 nm.

As taught by the '684 patent, and as exemplified in the examples below, not every combination of surface stabilizer and active agent will results in a stable nanoparticulate composition. It was surprisingly discovered that stable, nanoparticulate ebastine, or a salt or derivative thereof, formulations can be made.

Advantages of the nanoparticulate H1-histamine receptor antagonists, such as ebastine, formulations of the invention as compared to conventional non-nanoparticulate (microcrystalline or solubilized) formulations of the same H1-histamine receptor antagonist (*e*.*g*., ebastine) include, but are not limited to: (1) smaller tablet or other solid dosage form size; (2) smaller doses of drug required to obtain the same pharmacological effect; (3) increased bioavailability; (4) improved pharmacokinetic profiles; (5) substantially similar pharmacokinetic profiles of H1-histamine receptor antagonist compositions when administered in the fed versus the fasted state; (6) bioequivalency of H1-histamine receptor antagonist compositions when administered in the fed versus the fasted state; (7) increased rate of absorption of nanoparticulate compositions; (8) an increased rate of dissolution for the H1-histamine receptor antagonist compositions; and (9) the H1-histamine receptor antagonist compositions can be used in conjunction with other active agents useful in the treatment of seasonal and perennial allergic rhinitis and related diseases.

The present invention also includes nanoparticulate H1-histamine receptor antagonists, such as ebastine, or a salt or derivative thereof, compositions together with one or more non-toxic physiologically acceptable carriers, adjuvants, or vehicles, collectively referred to as carriers. The compositions can be formulated for parental injection (*e*.*g*., intravenous, intramuscular, or subcutaneous), oral administration in solid, liquid, or aerosol form, vaginal, nasal, rectal, ocular, local (powders, ointments, or drops), buccal, intracisternal, intraperitoneal, or topical administrations, and the like.

In some embodiments, a preferred dosage form of the invention is a solid dosage form, although any pharmaceutically acceptable dosage form can be utilized. Exemplary solid dosage forms include, but are not limited to, tablets, capsules, sachets, lozenges, powders, pills, or granules, and the solid dosage form can be, for example, a fast melt dosage form, controlled release dosage form, lyophilized dosage form, delayed release dosage form, extended release dosage form, pulsatile release dosage form, mixed immediate release and controlled release dosage form, or a combination thereof.

The present invention is described herein using several definitions, as set forth below and throughout the application.

The term "effective average particle size," as used herein, means that at least about 50% of the nanoparticulate H1-histamine receptor antagonist particles, such as ebastine, have a size of less than about 2000 nm (by weight or by other suitable measurement, such as by volume, number, etc.), when measured by, for example, sedimentation flow fractionation, photon correlation spectroscopy, light scattering, disk centrifugation, and other techniques known to those of skill in the art.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to stable nanoparticulate H1-histamine receptor antagonist, such as ebastine, particles, "stable" connotes, but is not limited to one or more of the following parameters: (1) the particles do not appreciably flocculate or agglomerate due to interparticle attractive forces or otherwise significantly increase in particle size over time; (2) the physical structure of the particles is not altered over time, such as by conversion from an amorphous phase to a crystalline phase; (3) the particles are chemically stable; and/or (4) where the H1-histamine receptor antagonist has not been subject to a heating step at or above the melting point of the H1-histamine receptor antagonist particles in the preparation of the nanoparticles of the present invention.

The term "conventional" or "non-nanoparticulate active agent" shall mean an active agent which is solubilized or which has an effective average particle size of greater than about 2000 nm. Nanoparticulate active agents as defined herein have an effective average particle size of less than about 2000 nm.

The phrase "poorly water soluble drugs" as used herein refers to those drugs that have a solubility in water of less than about 30 mg/ml, less than about 20 mg/ml, less than about 10 mg/ml, or less than about 1 mg/ml.

As used herein, the phrase "therapeutically effective amount" shall mean that drug dosage that provides the specific pharmacological response for which the drug is administered in a significant number of subjects in need of such treatment. It is emphasized that a therapeutically effective amount of a drug that is administered to a particular subject in a particular instance will not always be effective in treating the conditions/diseases described herein, even though such dosage is deemed to be a therapeutically effective amount by those of skill in the art.

### A. Characteristics of the Nanoparticulate Ebastine Compositions of the Invention

### 1. Increased Bioavailability

The H1-histamine receptor antagonist, such as ebastine, formulations of the invention are contemplated to exhibit increased bioavailability as compared to the same non-nanoparticulate H1-histamine receptor antagonist. Moreover, the compositions of the invention are expected to require smaller doses, and smaller tablet or other solid dosage form size as compared to prior conventional non-nanoparticulate formulations of the same H1-histamine receptor antagonist to achieve the same pharmacological effect.

The increased bioavailability is significant because it means that the nanoparticulate H1-histamine receptor antagonist dosage form will likely exhibit significantly greater drug absorption.

### 2. Improved Pharmacokinetic Profiles

The invention also contemplates H1-histamine receptor antagonist compositions having a desirable pharmacokinetic profile when administered to mammalian subjects. The desirable pharmacokinetic profile of the compositions comprising an H1-histamine receptor antagonist includes, but is not limited to: (1) a Cₘₐₓ for an H1-histamine receptor antagonist, such as ebastine, when assayed in the plasma of a mammalian subject following administration, that is preferably greater than the Cₘₐₓ for a non-nanoparticulate formulation of the same H1-histamine receptor antagonist, administered at the same dosage; and/or (2) an AUC for an H1-histamine receptor antagonist, such as ebastine, when assayed in the plasma of a mammalian subject following administration, that is preferably greater than the AUC for a non-nanoparticulate formulation of the same H1-histamine receptor antagonist, administered at the same dosage; and/or (3) a Tₘₐₓ for an H1-histamine receptor antagonist, such as ebastine, when assayed in the plasma of a mammalian subject following administration, that is preferably less than the Tₘₐₓ for a non-nanoparticulate formulation of the same H1-histamine receptor antagonist, administered at the same dosage. The desirable pharmacokinetic profile, as used herein, is the pharmacokinetic profile measured after the initial dose of a H1-histamine receptor antagonist, such as ebastine.

In one embodiment, a composition comprising a nanoparticulate H1-histamine receptor antagonist, such as ebastine, exhibits in comparative pharmacokinetic testing with a non-nanoparticulate formulation of the same H1-histamine receptor antagonist, administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ exhibited by the non-nanoparticulate H1-histamine receptor antagonist formulation.

In another embodiment, the composition comprising a nanoparticulate H1-histamine receptor antagonist, such as ebastine, exhibits in comparative pharmacokinetic testing with a non-nanoparticulate formulation of the same H1-histamine receptor antagonist, administered at the same dosage, a Cₘₐₓ which is at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by the non-nanoparticulate. H1-histamine receptor antagonist formulation..

In yet another embodiment, the composition comprising a nanoparticulate H1-histamine receptor antagonist, such as ebastine, exhibits in comparative pharmacokinetic testing with a non-nanoparticulate formulation of the same H1-histamine receptor antagonist, administered at the same dosage, an AUC which is at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate H1-histamine receptor antagonist formulation.

In one embodiment of the invention, the Tₘₐₓ of the H1-histamine receptor antagonist, such as ebastine, when assayed in the plasma of the mammalian subject, is less than about 6 to about 8 hours. In other embodiments of the invention, the Tₘₐₓ of the H1-histamine receptor antagonist is less than about 6 hours, less than about 5 hours, less than about 4 hours, less than about 3 hours, less than about 2 hours, less than about 1 hour, or less than about 30 minutes after administration.

The desirable pharmacokinetic profile, as used herein, is the pharmacokinetic profile measured after the initial dose of an H1-histamine receptor antagonist, such as ebastine. The compositions can be formulated in any way as described herein and as known to those of skill in the art.

### 3. The Pharmacokinetic Profiles of the H1-Histamine Receptor Antagonist Compositions of the Invention are not Affected by the Fed or Fasted State of the Subject Ingesting the Compositions

The invention encompasses an H1-histamine receptor antagonist, such as ebastine, composition wherein the pharmacokinetic profile of the H1-histamine receptor antagonist is not substantially affected by the fed or fasted state of a subject ingesting the composition. This means that there is no substantial difference in the quantity of drug absorbed (AUC), the rate of drug absorption (Cₘₐₓ), or the length of time to Cₘₐₓ (Tₘₐₓ), when the nanoparticulate H1-histamine receptor antagonist compositions are administered in the fed versus the fasted state.

### 4. Bioequivalency of H1-Histamine Receptor Antagonist Compositions of the Invention When Administered in the Fed Versus the Fasted State

The invention also encompasses a composition comprising a nanoparticulate H1-histamine receptor antagonist, such as ebastine, in which administration of the composition to a subject in a fasted state is bioequivalent to administration of the composition to a subject in a fed state.

The difference in absorption (AUC) or Cₘₐₓ of the H1-histamine receptor antagonist compositions of the invention (such as ebastine compositions), when administered in the fed versus the fasted state, preferably is less than about 100%, less than about 90%, less than about 80%, less than about 70%, less than about 60%, less than about 55%, less than about 50%, less than about 45%, less than about 40%, less than about 35%, less than about 30%, less than about 25%, less than about 20%, less than about 15%, less than about 10%, less than about 5%, or less than about 3%.

In one embodiment of the invention, the invention encompasses compositions comprising a nanoparticulate H1-histamine receptor antagonist, such as ebastine, wherein administration of the composition to a subject in a fasted state is bioequivalent to administration of the composition to a subject in a fed state, in particular as defined by Cₘₐₓ and AUC guidelines given by the U.S. Food and Drug Administration and the corresponding European regulatory agency (EMEA). Under U.S. FDA guidelines, two products or methods are bioequivalent if the 90% Confidence Intervals (Cl) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% Cl for AUC must be between 0.80 to 1.25 and the 90% Cl for Cₘₐₓ must between 0.70 to 1.43.

### 5. Dissolution Profiles of the H1-Histamine Receptor antagonist Compositions of the Invention

The compositions comprising a nanoparticulate H1-histamine receptor antagonist, such as ebastine, or a salt or derivative thereof, are proposed to have unexpectedly dramatic dissolution profiles. Rapid dissolution of an administered active agent is preferable, as faster dissolution generally leads to greater bioavailability and faster onset of action. To improve the dissolution profile and bioavailability of the H1-histamine receptor antagonist it would be useful to increase the drug's dissolution so that it could attain a level close to 100%.

The H1-histamine receptor antagonist, such as ebastine, compositions of the invention preferably have a dissolution profile in which within about 5 minutes at least about 20% of the composition is dissolved. In other embodiments of the invention, at least about 30% or at least about 40% of the H1-histamine receptor antagonist composition is dissolved within about 5 minutes. In yet other embodiments of the invention, preferably at least about 40%, at least about 50%, at least about 60%, at least about 70%, or at least about 80% of the H1-histamine receptor antagonist composition is dissolved within about 10 minutes. Finally, in another embodiment of the invention, preferably at least about 70%, at least about 80%, at least about 90%, or at least about 100% of the H1-histamine receptor antagonist composition is dissolved within 20 minutes.

Dissolution is preferably measured in a medium which is discriminating. A discriminating dissolution medium is one that will produce two very different dissolution curves for two products having very different dissolution profiles in gastric juices; *i.e*., the dissolution medium is predictive of the *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

### 6. Redispersibility of the H1-Histamine Receptor Antagonist Compositions of the Invention

An additional feature of the compositions comprising an H1-histamine receptor antagonist, such as ebastine or a salt or derivative thereof, is that the compositions redisperse such that the effective average particle size of the redispersed H1-histamine receptor antagonist particles is less than about 2 microns. This is significant, as upon administration, if the H1-histamine receptor antagonist particles of the compositions of the present invention agglomerated or did not redisperse to a substantially nanoparticulate size, then the dosage form may lose the benefits afforded by formulating the H1-histamine receptor antagonist into a nanoparticulate size.

This is because nanoparticulate active agent compositions benefit from the small particle size of the active agent. If the active agent does not disperse into the small particle sizes upon administration, then "clumps" or agglomerated active agent particles are formed, owing to the extremely high surface free energy of the nanoparticulate system and the thermodynamic driving force to achieve an overall reduction in free energy. With the formulation of such agglomerated particles, the bioavailability of the dosage form may fall well below that observed with the liquid dispersion form of the nanoparticulate active agent.

Moreover, the nanoparticulate H1-histamine receptor antagonist, such as ebastine, compositions of the invention exhibit dramatic redispersion of the nanoparticulate H1-histamine receptor antagonist particles upon administration to a mammal, such as a human or animal, as demonstrated by reconstitution/redispersion in a biorelevant aqueous media such that the effective average particle size of the redispersed H1-histamine receptor antagonist particles is less than about 2 microns. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1 M while fasted state intestinal fluid has an ionic strength of about 0.14. *See e.g*., Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (*i.e*., weak acids and corresponding salts of that acid), monoprotic and polyprotic electrolytes, etc.

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human Gl tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed particles of an H1-histamine receptor antagonist, such as ebastine or a salt or derivative thereof (redispersed in water, a biorelevant media, or any suitable redispersion media), have an effective average particle size of less than about less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods. Such methods suitable for measuring effective average particle size are known to a person of ordinary skill in the art.

Redispersibility can be tested using any suitable means known in the art. *See e*.*g*., the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### 7. Ebastine Compositions Used in Conjunction with Other Active Agents

The H1-histamine receptor antagonist, such as ebastine or a salt or derivative thereof, compositions of the invention can additionally comprise one or more compounds useful in the treatment of seasonal and perennial allergic rhinitis and related diseases, or the H1-histamine receptor antagonist compositions can be administered in conjunction with such a compound.

### B. Nanoparticulate Ebastine Compositions

The invention provides compositions comprising particles of at least one H1-histamine receptor antagonist, such as ebastine or a salt or derivative thereof, and at least one surface stabilizer. The surface stabilizers preferably are adsorbed on, or associated with, the surface of the ebastine particles. Surface stabilizers especially may physically adhere on, or associate with, the surface of the nanoparticulate H1-histamine receptor antagonists particles, but ideally do not chemically react with the particles of an H1-histamine receptor antagonists (such as ebastine) or itself. Individually adsorbed molecules of the surface stabilizer are essentially free of intermolecular cross-linkages.

The present invention also includes H1-histamine receptor antagonists such as ebastine, (or a salt or derivative thereof), compositions together with one or more non-toxic physiologically acceptable carriers, adjuvants, or vehicles, collectively referred to as carriers. The compositions can be formulated for parenteral injection (*e*.*g*., intravenous, intramuscular, or subcutaneous), oral administration in solid, liquid, or aerosol form, vaginal, nasal, rectal, ocular, local (powders, ointments or drops), buccal, intracisternal, intraperitoneal, or topical administration, and the like.

### 1. H1-histamine Receptor Antagonist Particles

The compositions of the invention comprise particles of at least one H1-histamine receptor antagonist, such as ebastine or a salt or derivative thereof. The particles can be in a crystalline phase, semi-crystalline phase, amorphous phase, semi-amorphous phase, or a combination thereof.

### 2. Surface Stabilizers

The choice of a surface stabilizer for an H1-histamine receptor antagonists such as ebastine is non-trivial. Accordingly, the present invention is directed to the surprising discovery that nanoparticulate H1-histamine receptor antagonist compositions can be made.

Combinations of more than one surface stabilizers can be used in the invention. Useful surface stabilizers which can be employed in the invention include, but are not limited to, known organic and inorganic pharmaceutical excipients. Such excipients include various polymers, low molecular weight oligomers, natural products, and surfactants. Exemplary surface stabilizers include nonionic and ionic (*e*.*g*., anionic, cationic, and zwitterionic) surfactants or compounds.

Representative examples of surface stabilizers include hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (*e.g*., macrogol ethers such as cetomacrogol 1000), polyoxyethylene castor oil derivatives, polyoxyethylene sorbitan fatty acid esters (*e*.*g*., the commercially available Tween^{®} products such as *e*.*g*., Tween 20^{®} and Tween 80^{®} (ICI Speciality Chemicals)); polyethylene glycols (*e.g*., Carbowaxs 3550^{®} and 934^{®} (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminium silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (*e*.*g*., Pluronics F68^{®} and F108^{®}, which are block copolymers of ethylene oxide and propylene oxide); poloxamines (*e*.*g*., Tetronic 908^{®}, also known as Poloxamine 908^{®}, which is a tetrafunctional block copolymer derived from sequential addition of propylene oxide and ethylene oxide to ethylenediamine (BASF Wyandotte Corporation, Parsippany, N.J.)); Tetronic 1508^{®} (T-1508) (BASF Wyandotte Corporation), Tritons X-200^{®}, which is an alkyl aryl polyether sulfonate (Rohm and Haas); Crodestas F-110^{®}, which is a mixture of sucrose stearate and sucrose distearate (Croda Inc.); p-isononylphenoxypoly-(glycidol), also known as Olin-IOG^{®} or Surfactant 10-G^{®} (Olin Chemicals, Stamford, CT); Crodestas SL-40^{®} (Croda, Inc.); and SA9OHCO, which is C₁₈H₃₇CH₂(CON(CH₃)-CH₂(CHOH)₄(CH₂OH)₂ (Eastman Kodak Co.); decanoyl-N-methylglucamide; n-decyl β-D-glucopyranoside; n-decyl β-D-maltopyranoside; n-dodecyl β-D-glucopyranoside; n-dodecyl β-D-maltoside; heptanoyl-N-methylglucamide; n-heptyl-β-D-glucopyranoside; n-heptyl β-D-thioglucoside; n-hexyl β-D-glucopyranoside; nonanoyl-N-methylglucamide; n-noyl β-D-glucopyranoside; octanoyl-N-methylglucamide; n-octyl-β-D-glucopyranoside; octyl β-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

If desirable, the nanoparticulate H1-histamine receptor antagonist, such as ebastine, compositions of the invention can be formulated to be phospholipid-free.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate.

Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C₁₂₋₁₅dimethyl hydroxyethyl ammonium chloride or bromide, coconut dimethyl hydroxyethyl ammonium chloride or bromide, myristyl trimethyl ammonium methyl sulphate, lauryl dimethyl benzyl ammonium chloride or bromide, lauryl dimethyl (ethenoxy)₄ ammonium chloride or bromide, N-alkyl (C₁₂₋₁₈)dimethylbenzyl ammonium chloride, N-alkyl (C₁₄₋₁₈)dimethyl-benzyl ammonium chloride, N-tetradecylidmethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C₁₂₋₁₄) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂, C₁₅, C₁₇ trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336™), POLYQUAT 10™, tetrabutylammonium bromide, benzyl trimethylammonium bromide, choline esters (such as choline esters of fatty acids), benzalkonium chloride, stearalkonium chloride compounds (such as stearyltrimonium chloride and Di-stearyldimonium chloride), cetyl pyridinium bromide or chloride, halide salts of quatemized polyoxyethylalkylamines, MIRAPOL™ and ALKAQUAT™ (Alkaril Chemical Company), alkyl pyridinium salts; amines, such as alkylamines, dialkylamines, alkanolamines, polyethylenepolyamines, N,N-dialkylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quartemary ammonium compounds of the formula NR₁R₂R₃R₄⁽⁺⁾. For compounds of the formula NR₁R₂R₃R₄⁽⁺⁾:
(i) none of R₁-R₄ are CH₃;
(ii) one of R₁-R₄ is CH₃;
(iii) three of R₁-R₄ are CH₃;
(iv) all of R₁-R₄ are CH₃;
(v) two of R₁-R₄ are CH₃, one of R₁-R₄ is C₆H₅CH₂, and one of R₁-R₄ is an alkyl chain of seven carbon atoms or less;
(vi) two of R₁-R₄ are CH₃, one of R₁-R₄ is C₆H₅CH₂, and one of R₁-R₄ is an alkyl chain of nineteen carbon atoms or more;
(vii) two of R₁-R₄ are CH₃ and one of R₁-R₄ is the group C₆H₅(CH₂)ₙ, where n>1;
(viii) two of R₁-R₄ are CH₃, one of R₁-R₄ is C₆H₅CH₂, and one of R₁-R₄ comprises at least one heteroatom;
(ix) two of R₁-R₄ are CH₃, one of R₁-R₄ is C₆H₅CH₂, and one of R₁-R₄ comprises at least one halogen;
(x) two of R₁-R₄ are CH₃, one of R₁-R₄ is C₆H₅CH₂, and one of R₁-R₄ comprises at least one cyclic fragment;
(xi) two of R₁-R₄ are CH₃ and one of R₁-R₄ is a phenyl ring; or
(xii) two of R₁-R₄ are CH₃ and two of R₁-R₄ are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride(Quaternium-14), Quatemium-22, Quatemium-26, Quatemium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

In some embodiments, the surface stabilizers may be a copovidone (*e*.*g*., Plasdone S630, which is a random copolymer of vinyl acetate and vinyl pyrrolidone) and/or docusate sodium.

The surface stabilizers are commercially available and/or can be prepared by techniques known in the art. Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated by reference.

### 3. Other Pharmaceutical Excipients

Pharmaceutical compositions according to the invention may also comprise one or more binding agents, filling agents, lubricating agents, suspending agents, sweeteners, flavoring agents, preservatives, buffers, wetting agents, disintegrants, effervescent agents, and other excipients. Such excipients are known in the art.

Examples of filling agents include lactose monohydrate, lactose anhydrous, and various starches; examples of binding agents include various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC™).

Suitable lubricants, including agents that act on the flowability of the powder to be compressed, may include colloidal silicon dioxide, such as Aerosil^{®} 200, talc, stearic acid, magnesium stearate, calcium stearate, and silica gel.

Examples of sweeteners may include any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives include potassium sorbate, methylparaben, propylparaben, benzoic acid and its salts, other esters of parahydroxybenzoic acid such as butylparaben, alcohols such as ethyl or benzyl alcohol, phenolic compounds such as phenol, or quartemary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, com starch, potato starch, maize starch, and modified starches; croscarmellose sodium, cross-povidone, sodium starch glycolate, and mixtures thereof.

Examples of effervescent agents include effervescent couples such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### 4. Nanoparticulate H1-Histamine Receptor Antagonist Particle Size

The compositions of the invention comprise nanoparticulate H1-histamine receptor antagonist, such as ebastine (or a salt or derivative thereof), particles which have an effective average particle size of less than about 2000 nm (*i*.*e*., 2 microns), less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

By "an effective average particle size of less than about 2000 nm" it is meant that at least 50% of the H1-histamine receptor antagonist, such as ebastine, particles have a particle size of less than the effective average, by weight (or by other suitable measurement techniques, such as by volume, number, etc.), *i*.*e*., less than about 2000 nm, 1900 nm, 1800 nm, *etc*., when measured by the above-noted techniques. Preferably, at least about 60%, at least about 70%, at least about 80% at least about 90%, or at least about 95% of the H1-histamine receptor antagonist, such as ebastine, particles have a particle size of less than the effective average, *i*.*e*., less than about 2000 nm, 1900 nm, 1800 nm, 1700 nm, *etc.*

In the present invention, the value for D50 of a nanoparticulate H1-histamine receptor antagonist, such as ebastine composition is the particle size below which 50% of the H1-histamine receptor antagonist particles fall, by weight. Similariy, D90 is the particle size below which 90% of the H1-histamine receptor antagonist particles fall, by weight.

### 5. Concentration of H1-Histamine Receptor Antagonist and Surface Stabilizers

The relative amounts of H1-histamine receptor antagonist, such as ebastine or a salt or derivative thereof, and one or more surface stabilizers may vary. The optimal amount of the individual components can depend, for example, upon the particular H1-histamine receptor antagonist selected, the hydrophilic lipophilic balance (HLB), melting point, and the surface tension of water solutions of the stabilizer, etc.

The concentration of the H1-histamine receptor antagonist (such as ebastine) may be present from about 99.5% to about 0.001%, from about 95% to about 0.1%, or from about 90% to about 0.5%, by weight, based on the total combined dry weight of the H1-histamine receptor antagonist and at least one surface stabilizer, not including other excipients.

The concentration of the at least one surface stabilizer may be present from about 0.5% to about 99.999%, from about 5.0% to about 99.9%, or from about 10% to about 99.5%, by weight, based on the total combined dry weight of the H1-histamine receptor antagonist and at least one surface stabilizer, not including other excipients.

### 6. Exemplary Nanoparticulate Ebastine Tablet Formulations

Several exemplary ebastine tablet formulations are given below. These examples are not intended to limit the claims in any respect, but rather to provide exemplary tablet formulations of ebastine which can be utilized in the methods of the invention. Such exemplary tablets may also include a coating agent.

| **Exemplary Nanoparticulate** **Ebastine Tablet Formulation #1** | |
|---|---|
| **Component** | **g/Kg** |
| Ebastine | about 50 to about 500 |
| Hypromellose, USP | about 10 to about 70 |
| Docusate Sodium, USP | about 1 to about 10 |
| Sucrose, NF | about 100 to about 500 |
| Sodium Lauryl Sulfate, NF | about 1 to about 40 |
| Lactose Monohydrate, NF | about 50 to about 400 |
| Silicified Microcrystalline Cellulose | about 50 to about 300 |
| Crospovidone, NF | about 20 to about 300 |
| Magnesium Stearate, NF | about 0.5 to about 5 |

| **Exemplary Nanoparticulate** **Ebastine Tablet Formulation #2** | |
|---|---|
| **Component** | **g/Kg** |
| Ebastine | about 100 to about 300 |
| Hypromellose, USP | about 30 to about 50 |
| Docusate Sodium, USP | about 0.5 to about 10 |
| Sucrose, NF | about 100 to about 300 |
| Sodium Lauryl Sulfate, NF | about 1 to about 30 |
| Lactose Monohydrate, NF | about 100 to about 300 |
| Silicified Microcrystalline Cellulose | about 50 to about 200 |
| Crospovidone, NF | about 50 to about 200 |
| Magnesium Stearate, NF | about 0.5 to about 5 |

| **Exemplary Nanoparticulate** **Ebastine Tablet Formulation #3** | |
|---|---|
| **Component** | **g/Kg**' |
| Ebastine | about 200 to about 225 |
| Hypromellose, USP | about 42 to about 46 |
| Docusate Sodium, USP | about 2 to about 6 |
| Sucrose, NF | about 200 to about 225 |
| Sodium Lauryl Sulfate, NF | about 12 to about 18 |
| Lactose Monohydrate, NF | about 200 to about 205 |
| Silicified Microcrystalline Cellulose | about 130 to about 135 |
| Crospovidone, NF | about 112 to about 118 |
| Magnesium Stearate, NF | about 0.5 to about 3 |

| **Exemplary Nanoparticulate** **Ebastine Tablet Formulation #4** | |
|---|---|
| **Component** | **g/Kg** |
| Ebastine | about 119 to about 224 |
| Hypromellose, USP | about 42 to about 46 |
| Docusate Sodium, USP | about 2 to about 6 |
| Sucrose, NF | about 119 to about 224 |
| Sodium Lauryl Sulfate, NF | about 12 to about 18 |
| Lactose Monohydrate, NF | about 119 to about 224 |
| Silicified Microcrystalline Cellulose | about 129 to about 134 |
| Crospovidone, NF | about 112 to about 118 |
| Magnesium Stearate, NF | about 0.5 to about 3 |

### C. Methods of Making Nanoparticulate H1-Histamine Receptor Antagonist Compositions

The nanoparticulate H1-histamine receptor antagonist, such as ebastine (or a salt or derivative thereof), compositions can be made using, for example, milling, homogenization, supercritical particle generation, precipitation, freezing, or template emulsion techniques. Exemplary methods of making nanoparticulate compositions are described in the '684 patent. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation of Nanoparticulate Pharmaceutical Agents with Crystal Growth Modifiers;" U.S. Patent No. 5,662,883 for "Co-Microprecipitation of Nanoparticulate Pharmaceutical Agents with Crystal Growth Modifiers;" U.S. Patent No. 5,560,932 for "Microprecipitation of Nanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated by reference.

The resultant nanoparticulate H1-histamine receptor antagonist compositions or dispersions can be utilized in solid or liquid dosage formulations, such as liquid dispersions, gels, aerosols, ointments, creams, controlled release formulations, fast melt formulations, lyophilized formulations, tablets, capsules, delayed release formulations, extended release formulations, pulsatile release formulations, mixed immediate release and controlled release formulations, etc.

### 1. Milling to Obtain Nanoparticulate Ebastine Dispersions

Milling an H1-histamine receptor antagonist, such as ebastine or a salt or derivative thereof, to obtain a nanoparticulate dispersion comprises dispersing the H1-histamine receptor antagonist particles in a liquid dispersion medium in which the H1-histamine receptor antagonist is poorly soluble, followed by applying mechanical means in the presence of grinding media to reduce the particle size of the H1-histamine receptor antagonist to the desired effective average particle size. The dispersion medium can be, for example, water, safflower oil, ethanol, t-butanol, glycerin, polyethylene glycol (PEG), hexane, or glycol. In some embodiments, a preferred dispersion medium is water.

The H1-histamine receptor antagonist particles can be reduced in size in the presence of at least one surface stabilizer. Alternatively, H1-histamine receptor antagonist particles can be contacted with one or more surface stabilizers after attrition. Other compounds, such as a diluent, can be added to the H1-histamine receptor antagonist /surface stabilizer composition during the size reduction process Dispersions can be manufactured continuously or in a batch mode.

### 2. Precipitation to Obtain Nanoparticulate H1-Histamine Receptor Antagonist Compositions

Another method of forming the desired nanoparticulate H1-histamine receptor antagonist, such as ebastine or a salt or derivative thereof, composition is by microprecipitation. This is a method of preparing stable dispersions of poorly soluble active agents in the presence of one or more surface stabilizers and one or more colloid stability enhancing surface active agents free of any trace toxic solvents or solubilized heavy metal impurities. Such a method comprises, for example: (1) dissolving the H1-histamine receptor antagonist in a suitable solvent; (2) adding the formulation from step (1) to a solution comprising at least one surface stabilizer; and (3) precipitating the formulation from step (2) using an appropriate non-solvent. The method can be followed by removal of any formed salt, if present, by dialysis or diafiltration and concentration of the dispersion by conventional means.

### 3. Homogenization to Obtain Nanoparticulate H1-Histamine Receptor Antagonist Compositions

Exemplary homogenization methods of preparing active agent nanoparticulate compositions are described in U.S. Patent No. 5,510,118, for "Process of Preparing Therapeutic Compositions Containing Nanoparticles." Such a method comprises dispersing particles of an ebastine, (or a salt or derivative thereof), in a liquid dispersion medium, followed by subjecting the dispersion to homogenization to reduce the particle size of an H1-histamine receptor antagonist to the desired effective average particle size. The particles can be reduced in size in the presence of at least one surface stabilizer. Alternatively, the H1-histamine receptor antagonist particles can be contacted with one or more surface stabilizers either before or after attrition. Other compounds, such as a diluent, can be added to the H1-histamine receptor antagonist /surface stabilizer composition either before, during, or after the size reduction process. Dispersions can be manufactured continuously or in a batch mode.

### 4. Cryogenic Methodologies to Obtain Nanoparticulate H1-Histamine Receptor Antagonist Compositions

Another method of forming the desired nanoparticulate H1-histamine receptor antagonist such as ebastine (or a salt or derivative thereof), composition is by spray freezing into liquid (SFL). This technology comprises an organic or organoaqueous solution of H1-histamine receptor antagonist with stabilizers, which is injected into a cryogenic liquid, such as liquid nitrogen. The droplets of the ebastine solution freeze at a rate sufficient to minimize crystallization and particle growth, thus formulating nanostructured ebastine particles. Depending on the choice of solvent system and processing conditions, the nanoparticulate H1-histamine receptor antagonist particles can have varying particle morphology. In the isolation step, the nitrogen and solvent are removed under conditions that avoid agglomeration or ripening of the H1-histamine receptor antagonist particles.

As a complementary technology to SFL, ultra rapid freezing (URF) may also be used to created equivalent nanostructured H1-histamine receptor antagonist particles with greatly enhanced surface area.

URF comprises an organic or organoaqueous solution of H1-histamine receptor antagonist with stabilizers onto a cryogenic substrate.

### 5. Emulsion Methodologies to Obtain Nanoparticulate H1-Histamine Receptor Antagonist Compositions

Another method of forming the desired nanoparticulate antagonist, such as ebastine, composition is by template emulsion. Template emulsion creates nanostructured H1-histamine receptor antagonist derivative particles with controlled particle size distribution and rapid dissolution performance. The method comprises an oil-in-water emulsion that is prepared, then swelled with a non-aqueous solution comprising the H1-histamine receptor antagonist and stabilizers. The particle size distribution of H1-histamine receptor antagonist particles is a direct result of the size of the emulsion droplets prior to loading with the H1-histamine receptor antagonist, a property which can be controlled and optimized in this process. Furthermore, through selected use of solvents and stabilizers, emulsion stability is achieved with no or suppressed Ostwald ripening. Subsequently, the solvent and water are removed, and the stabilized nanostructured H1-histamine receptor antagonist particles are recovered. Various H1-histamine receptor antagonist particles morphologies can be achieved by appropriate control of processing conditions.

### D. Methods of Using the Nanoparticulate Ebastine Compositions of the Invention

The invention provides a method of increasing bioavailability (*e*.*g*., increasing the plasma levels) of an H1-histamine receptor antagonist such as ebastine (or a salt or derivative thereof), in a subject. Such a method comprises orally administering to a subject an effective amount of a composition comprising a nanoparticulate H1-histamine receptor antagonist. The nanoparticulate H1-histamine receptor antagonist such as ebastine, composition, in accordance with standard pharmacokinetic practice, would exhibit a bioavailability that is about 50% greater than a conventional dosage form, about 40% greater, about 30% greater, about 20% or about 10% greater. Additionally, the compositions when tested in fasting subjects in accordance with standard pharmacokinetic practice, are proposed to produces a maximum blood plasma concentration profile in less than about 6 hours, less than about 5 hours, less than about 4 hours, less than about 3 hours, less than about 2 hours, less than about 1 hour or less than about 30 minutes after the initial dose of the composition.

The compositions of the invention may be useful in the treatment of seasonal and perennial allergic rhinitis, idiopathic chronic urticaria and related diseases.

The H1-histamine receptor antagonist, such as ebastine (or a salt or derivative thereof), compounds of the invention can be administered to a subject via any conventional means including, but not limited to, orally, rectally, ocularly, parenterally (*e*.*g*., intravenous, intramuscular, or subcutaneous), intracistemally, pulmonary, intravaginally, intraperitoneally, locally (*e*.*g*., powders, ointments or drops), or as a buccal or nasal spray. As used herein, the term "subject" is used to mean an animal, preferably a mammal, including a human or non-human. The terms patient and subject may be used interchangeably.

Compositions suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents, or vehicles including water, ethanol, polyols (propyleneglycol, polyethyleneglycol, glycerol, and the like), suitable mixtures thereof, vegetable oils (such as olive oil) and injectable organic esters such as ethyl oleate. Proper fluidity can be maintained, for example, by the use of a coating such as lecithin, by the maintenance of the required particle size in the case of dispersions, and by the use of surfactants.

The nanoparticulate H1-histamine receptor antagonist, such as ebastine (or a salt or derivative thereof) compositions may also contain adjuvants such as preserving, wetting, emulsifying, and dispensing agents. Prevention of the growth of microorganisms can be ensured by various antibacterial and antifungal agents, such as parabens, chlorobutanol, Phenol, sorbic acid, and the like. It may also be desirable to include isotonic agents, such as sugars, sodium chloride, and the like. Prolonged absorption of the injectable pharmaceutical form can be brought about by the use of agents delaying absorption, such as aluminum monostearate and gelatin.

Solid dosage forms for oral administration include, but are not limited to, capsules, tablets, pilis, powders, and granules. In such solid dosage forms, the active agent is admixed with at least one of the following: (a) one or more inert excipients (or carriers), such as sodium citrate or dicalcium phosphate; (b) fillers or extenders, such as starches, lactose, sucrose, glucose, mannitol, and silicic acid; (c) binders, such as carboxymethylcellulose, alignates, gelatin, polyvinylpyrrolidone, sucrose, and acacia; (d) humectants, such as glycerol; (e) disintegrating agents, such as agar-agar, calcium carbonate, potato or tapioca starch, alginic acid, certain complex silicates, and sodium carbonate; (f) solution retarders, such as paraffin; (g) absorption accelerators, such as quaternary ammonium compounds; (h) wetting agents, such as cetyl alcohol and glycerol monostearate; (i) adsorbents, such as kaolin and bentonite; and (j) lubricants, such as talc, calcium stearate, magnesium stearate, solid polyethylene glycols, sodium lauryl sulfate, or mixtures thereof. For capsules, tablets, and pills, the dosage forms may also comprise buffering agents.

Liquid dosage forms for oral administration include pharmaceutically acceptable emulsions, solutions, suspensions, syrups, and elixirs. In addition to an H1-histamine receptor antagonist such as ebastine, the liquid dosage forms may comprises inert diluents commonly used in the art, such as water or other solvents, solubilizing agents, and emulsifiers. Exemplary emulsifiers are ethyl alcohol, isopropyl alcohol, ethyl carbonate, ethyl acetate, benzyl alcohol, benzyl benzoate, propyleneglycol, 1,3-butyleneglycol, dimethylformamide, oils, such as cottonseed oil, groundnut oil, com germ oil, olive oil, castor oil, and sesame oil, glycerol, tetrahydrofurfuryl alcohol, polyethyleneglycols, fatty acid esters of sorbitan, or mixtures of these substances, and the like.

Besides such inert diluents, the composition can also include adjuvants, such as wetting agents, emulsifying and suspending agents, sweetening, flavoring, and perfuming agents.

"Therapeutically effective amount" as used herein with respect to, for example, an ebastine dosage shall mean that dosage that provides the specific pharmacological response for which an ebastine is administered in a significant number of subjects in need of such treatment It is emphasized that "therapeutically effective amount," administered to a particular subject in a particular instance will not always be effective in treating the diseases described herein, even though such dosage is deemed a "therapeutically effective amount" by those skilled in the art. It is to be further understood that ebastine dosages are, in particular instances, measured as oral dosages, or with reference to drug levels as measured in blood.

One of ordinary skill will appreciate that effective amounts of an H1-histamine receptor antagonist such as ebastine can be determined empirically and can be employed in pure form or, where such forms exist, in pharmaceutically acceptable salt; ester, or prodrug form. Actual dosage levels of an H1-histamine receptor antagonist such as ebastine in the nanoparticulate compositions of the invention may be varied to obtain an amount of an H1-histamine receptor antagonist such as ebastine that is effective to obtain a desired therapeutic response for a particular composition and method of administration. The selected dosage level therefore depends upon the desired therapeutic effect, the route of administration, the potency of the administered H1-histamine receptor antagonist such as ebastine, the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

### E. Examples

The following examples are provided to illustrate the present invention. It should be understood, however, that the invention is not to be limited to the specific conditions or details described in these examples. Throughout the specification, any and all references to a publicly available document, including a U.S. patent, are specifically incorporated by reference.

### Example 1

The purpose of this example was to prepare various nanoparticulate formulations of ebastine using different surface stabilizers.

Eleven different nanoparticulate formulations of ebastine were prepared using the following parameters. The preparations were formulated and milled using a NanoMill-01, with a 10-ml Chamber, (NanoMill Systems, King of Prussia, Philadelphia, described in U.S. Pat. No. 6,431,478). The attrition media was PolyMill 500 (500 micron polymeric attrition media) with media loading at 89%. All sample were milled at a mill speed of 2500 rpm for 60 minutes, except for Sample No. 2 which was milled at 3500 rpm for 60 minutes, and Sample No. 3 which was milled at 2500 rpm for 90 minutes.

Particle size measurement medium for all samples was MilliQ water. The nanoparticulate compositions were observed with a Lecia DM5000B microscope and Lecia CTR 5000 light source (Laboratory Instruments & Supplies (I) Ltd., Ashboume CO MEATH ROI) and composition characteristics were noted. Particle size was measured using the Horiba LA-910 Light Scattering Particle Size Analyzer (Particular Sciences, Hatton Derbyshire, England).

Results are shown in Tables 1 and 2, below. Table 1 notes Sample Number, the formulation of that sample, microscopy observations and a designation of whether the formulation was successful (*e*.*g*., average effective particle size as determined by D50 is less than about 2000 nm without sonication). "Y" indicates "yes," the formulation was successful. "N" indicates "no," the formulation was not successful.

Table 2 provides a statistical analysis of the compositions and indicates, for each formulation, mean, mode and median particle size either before or after a 60 second sonication. "Y" indicates 60 second sonication, "N" indicates no sonication. D50/nm indicates that 50% of the particles are less than the noted value by weight. D90 indicates that 90% of the particles are below the noted value by weight, and D95 indicates that 95% of the particles are below the noted value by weight.

| **TABLE 1** | | | |
|---|---|---|---|
| **Sample No.** | **Formulation (all by (w/w)** | **Microscopy Observations** | **Success?** |
| 1 | Ebastine, 5% HPC-SL, 2% Deionised Water, 93% | Microscopy showed the sample to be well dispersed with nanoparticles of ebastine clearly visible. Brownian motion was also clearly observed. There was no sign of ebastine particle flocculation or ebastine crystal growth. However there were signs of unmilled drug particles throughout the sample. | Y |
| 2 | Ebastine, 5% Plasdone S-630, 1.25% Sodium Lauryl Sulfate, 0.05% Deionised Water, 93.7% | Microscopy showed the sample to be well dispersed with nanoparticles of ebastine clearly visible. However, there was a large number of unmilled drug particles throughout the sample. There was no sign of ebastine crystal growth. | Y |
| 3 | Ebastine, 5% Lutrol F68, 1.25% Docusate sodium, 0.05% Deionised Water, 93.7% | Microscopy showed the sample to be well dispersed with nanoparticles of ebastine visible. Brownian motion was also observed. There was some evidence of unmilled drug particles and some slight ebastine particle flocculation as seen on the microphotographs. There was no signs of ebastine crystal growth | Y |
| 4 | Ebastine, 5% Tween 80, 1.5% Lecithin, 0.05% Deionised Water, 93.45% | Microscopy showed that some nanoparticles of ebastine were visible. Larger ebastine particles were also observed throughout the sample which may indicate the presence of ebastine crystal growth or some unmilled drug particles. There was no evidence of Brownian motion. | N |
| 5 | Ebastine, 5% Pharmacoat 603, 1.25% Docusate sodium, 0.05% Deionised Water, 93.7% | The sample from the microscopy showed to be reasonably well dispersed with signs of unmilled drug particle present. Particle size analysis D50 data is below 2000 nm. No signs of ebastine particle flocculation were visible and nanoparticles of ebastine were clearly visible as well as Brownian motion. | Y |
| 6 | Ebastine, 5% Tyloxapol, 1.25% Deionised Water, 93.75% | The sample appeared to be well dispersed with nanoparticles of ebastine clearly visible. There was no sign of ebastine particle flocculation or ebastine crystal growth. There were some small particles evident throughout the sample which may indicate some partially milled drug particles. | Y |
| 7 | Ebastine, 5% Plasdone K17, 1.25% Benzalkonium chloride, 0.05% Deionised Water, 93.7% | Microscopy showed flocculation of ebastine particles with a large number of large ebastine particles spread throughout the sample. These may have been unmilled drug particles or ebastine crystal growth. There were nanoparticles of ebastine clearly visible and some Brownian motion. | Y |
| 8 | Ebastine, 5% Plasdone K29/32, 1.25% Sodium Lauryl Sulfate, 0.05% Deionised Water, 93.7% | Nanoparticles of ebastine and Brownian motion were observed. However, there was a large number of unmilled drug particles throughout the sample. Flocculation was also observed throughout the sample. | Y |
| 9 | Ebastine, 5% Tween 80, 1.5% Deionised water, 93.5% | Some nanoparticles of ebastine were visible and Brownian motion was clearly evident under the microscope. Larger particles were also observed which may indicate the presence of ebastine crystal growth or partially milled material. | Y |
| 10 | Ebastine, 5% Plasdone C-15, 1.25% Deoxycholate sodium, 0.05% Deionised Water, 93.7% | Some nanoparticles were observed and some Brownian motion was evident. Some rod-like crystals and agglomeration were also observed. | N |
| 11 | Ebastine, 5% Lutrol F108 1.5% Deionised Water, 93.5% | Some nanoparticles of ebastine were observed together with Brownian motion. However, some rod-like crystals were also noted. Also, there was some evidence of ebastine particle agglomeration present in the sample. | Y |

| **TABLE 2** | | | | | | | |
|---|---|---|---|---|---|---|---|
| **Sample No.** | **Mean/ nm** | **D50/ nm** | **D90/ nm** | **D95/ nm** | **Mode /nm** | **Median/ nm** | **Sonication 60s.** |
| 1 | 465 | 319 | 602 | 1253 | 314 | 319 | N |
| | 465 | 315 | 584 | 1288 | 314 | 315 | Y |
| 2 | 1369 | 438 | 3945 | 5592 | 279 | 438 | N |
| | 1360 | 431 | 3927 | 5518 | 280 | 431 | Y |
| 3 | 2132 | 339 | 8186 | 15522 | 316 | 339 | N |
| | 348 | 322 | 500 | 583 | 317 | 322 | Y |
| 4 | 3484 | 3084 | 6947 | 8319 | 4761 | 3084 | N |
| | 1355 | 1209 | 2423 | 2962 | 1405 | 1209 | Y |
| 5 | 859 | 389 | 2217 | 3592 | 319 | 389 | N |
| | 869 | 384 | 2312 | 3692 | 318 | 384 | Y |
| 6 | 187 | 181 | 240 | 258 | 182 | 181 | N |
| | 181 | 176 | 231 | 253 | 166 | 176 | Y |
| 7 | 458 | 414 | 727 | 859 | 414 | 414 | N |
| | 458 | 416 | 722 | 850 | 415 | 416 | Y |
| 8 | 4977 | 687 | 17610 | 22653 | 476 | 687 | N |
| | 716 | 525 | 1182 | 1779 | 477 | 525 | Y |
| 9 | 1445 | 1342 | 2588 | 3118 | 1612 | 1342 | N |
| | 1464 | 1360 | 2591 | 3118 | 1611 | 1360 | Y |
| 10 | 5560 | 4970 | 11784 | 14238 | 8189 | 4970 | N |
| | 1357 | 945 | 2898 | 4002 | 1232 | 945 | Y |
| 11 | 1379 | 1253 | 2695 | 3260 | 1622 | 1253 | N |
| | 1410 | 1293 | 2723 | 3287 | 1622 | 1293 | Y |

## Claims

1. A stable nanoparticulate ebastine, or a salt thereof, composition comprising:
(a) particles of ebastine or a salt thereof having an effective average particle size of less than about 2000 nm; and
(b) at least one surface stabilizer.

2. The composition of claim 1, wherein the ebastine is in a crystalline phase, an amorphous phase, a semi-crystalline phase, a semi amorphous phase, or mixtures thereof.

3. The composition of claim 1 or claim 2, wherein the effective average particle size of the particles of ebastine or a salt there of is selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than abut 400 nm, less than about 300 nm, less than about 250 nm, less than about 200 nm, less than about 100 nm, less than about 75 nm, and less than about 50 nm.

4. The composition of any one of claims 1 to 3, wherein the nanoparticulate ebastine composition has improved bioavailability as compared to conventional ebastine tablets.

5. The composition of any one of claims 1 to 4, wherein the composition is formulated:
(a) for administration selected from the group consisting of oral, pulmonary, intravenous, rectal, opthalmic, colonic, parenteral, intracisternal, intravaginal, intraperitoneal, local, buccal, nasal, and topical administration;
(b) into a dosage form selected from the group consisting of liquid dispersions, gels, aerosols, ointments, creams, tablets, sachets and capsules;
(c) into a dosage form selected from the group consisting of lyophilized formulations, fast melt formulations, controlled release formulations, delayed release formulations, extended release formulations, pulsatile release formulations, and mixed immediate release and controlled release formulations; or
(d) any combination of (a), (b), and (c).

6. The composition of any one of claims 1 to 5, wherein the composition further comprises one or more pharmaceutically acceptable excipients, carriers, or a combination thereof.

7. The composition of any one of claims 1 to 6, wherein:
(a) the amount of ebastine is selected from the group consisting of from abut 99.5% to about 0.001%, from about 95% to about 0.1%, and from about 90% to about 0.5%, by weight, based on the total combined weight of ebastine and at least one surface stabilizer, not including other excipients;
(b) at least one surface stabilizer is present in an amount selected from the group consisting 0.01% to about 99.5% by weight, from about 0.1% to about 95% by weight, from about 0.5% to about 90% by weight, from about 5.0% to about 99.9% by weight, and from about 10% to about 99.5% by weight, based on the total combined dry weight of ebastine and at least one surface stabilizer, not including other excipients; or
(c) a combination of (a) and (b).

8. The composition of any one of claims 1 to 7, comprising at least one primary surface stabilizer and at least one secondary surface stabilizer.

9. The composition of any one of claims 1 to 8, wherein at least one surface stabilizer is selected from the group consisting of a non-ionic surface stabilizer, an ionic surface stabilizer, an anionic surface stabilizer, a cationic surface stabilizer, and a zwitterionic surface stabilizer.

10. The composition of any one of claims 1 to 9, wherein at least one surface stabilizer is selected from the group consisting of cetyl pyridinium chloride, gelatin, casein, phosphatides, dextran, glycerol, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, dodecyl trimethyl ammonium bromide, polyoxyethylene stearates, colloidal silicon dioxide, phosphates, sodium dodecylsulfate, carboxymethylcellulose calcium, hydroxypropyl celluloses, hypromellose, carboxymethylcellulose sodium, methylcellulose, hydroxyethylcellulose, hypromellose phthalate, noncrystalline cellulose, magnesium aluminum silicate, triethanolamine, polyvinyl alcohol, polyvinylpyrrolidone, 4-(1,1,3,3-tetramethylbutyl)-phenol polymer with ethylene oxide and formaldehyde, poloxamers; poloxamines, a charged phospholipide, dioctylsulfosuccinate, dialkylesters of sodium sulfosuccinic acid, sodium lauryl sulfate, alkyl aryl polyether sulfonates, mixtures of sucrose stearate and sucrose distearate, C₁₈H₃₇CH₂C(O)N(CH₃)-CH₂(CHOH)₄(CH₂OH)₂, p-isononylphenoxypoly-(glycidol), decanoyl-N-methylglucamide; n-decyl β-D-glucopyranoside; n-decyl β-D-maltopyranoside; n-dodecyl β-D-glucopyranoside; n-dodecyl β-D-maltoside; heptanoyi-N-methylglucamide; n-heptyl-β-D-glucopyranoside; n-heptyl β-D-thioglucoside; n-hexyl β-D-glucopyranoside; nonanoyl-N-methylglucamide; n-nonyl β-D-glucopyranoside; octanoyl-N-methylglucamide; n-octyl-β-D-glucopyranoside; octyl β-D-thioglucopyranoside; lysozyme, PEG-phospholipid, PEG-cholesterol, PEG-cholesterol derivative, PEG-vitamin A, PEG-vitamin E, random copolymers of vinyl acetate and vinyl pyrrolidone, a cationic polymer, a cationic biopolymer, a cationic polysaccharide, a cationic cellulosic, a cationic alginate, a cationic nonpolymeric compound, a cationic phospholipid, cationic lipids, polymethylmethacrytate trimethylammonium bromide, sulfonium compounds, polyvinylpyrrolidone-2-dimethylaminoethyl methacrylate dimethyl sulfate, hexadecyltrimethyl ammonium bromide, phosphonium compounds, quartemary ammonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium chloride bromide, C₁₂₋₁₅dimethyl hydroxyethyl ammonium chloride, C₁₂₋₁₅dimethyl hydroxyethyl ammonium chloride bromide, coconut dimethyl hydroxyethyl ammonium chloride, coconut dimethyl hydroxyethyl ammonium bromide, myristyl trimethyl ammonium methyl sulphate, lauryl dimethyl benzyl ammonium chloride, lauryl dimethyl benzyl ammonium bromide, lauryl dimethyl (ethenoxy)₄ ammonium chloride, lauryl dimethyl (ethenoxy)₄ ammonium bromide, N-alkyl (C₁₂₋₁₈)dimethylbenzyl ammonium chloride, N-alkyl (C₁₄₋₁₈)dimethyl-benzyl ammonium chloride, N-tetradecydimethylbenzyl ammonium chloride monohydrate, dimethyl didecyl ammonium chloride, N-alkyl and (C₁₂₋₁₄) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkylamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium chloride monohydrate, N-alkyl(C₁₂₋₁₄) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C₁₂ trimethyl ammonium bromides, C₁₅ trimethyl ammonium bromides, C₁₇ trimethyl ammonium bromides, dodecylbenzyl trimethyl ammonium chloride, polydiallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT 10™, tetrabutylammonium bromide, benzyl trimethylammonium bromide, choline esters, benzalkonium chloride, stearalkonium chloride compounds, cetyl pyridinium bromide, halide salts of quaternized polyoxyethylalkylamines, MIRAPOL™, ALKAQUAT™, alkyl pyridinium salts; amines, amine salts, amine oxides, imide azolinium salts, protonated quaternary acrylamides, methylated quaternary polymers; and cationic guar.

11. The composition of any one of claims 1 to 10, additionally comprising one or more active agents useful for the treatment of seasonal and perennial allergic rhinitis and related diseases.

12. The composition of any one of claims 1 to 11, wherein:
(a) upon administration to a mammal the particles of ebastine or a salt thereof redisperse such that the particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm;
(b) the composition redisperses in a biorelevant medium such that the ebastine particles have an effective average particle size selected from the group consisting of less than about 2 microns, less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 600 nm, less than about 500 nm, less than about 400 nm, less than about 300 nm, less than about 250 nm, less than about 200 nm, less than about 100 nm, less than about 75 nm, and less than about 50 nm; or
(c) a combination of (a) and (b).

13. The composition of claim 12, wherein the biorelevant medium is selected from the group consisting of water, aqueous electrolyte solutions, aqueous solutions of a salt, aqueous solutions of an acid, aqueous solutions of a base, and combinations thereof.

14. The composition of any one of claims 1 to 13, wherein:
(a) the Tₘₐₓ of the nanoparticulate ebastine composition, when assayed in the plasma of a mammalian subject following administration, is less than the Tₘₐₓ for a non-nanoparticulate composition of the same ebastine, administered at the same dosage;
(b) the Cₘₐₓ of the nanoparticulate ebastine composition, when assayed in the plasma of a mammalian subject following administration, is greater than the Cₘₐₓ for a non-nanoparticulate composition of the same ebastine, administered at the same dosage;
(c) the AUC of the nanoparticulate ebastine composition, when assayed in the plasma of a mammalian subject following administration, is greater than the AUC for a non-nanoparticulate composition of the same ebastine, administered at the same dosage; or
(d) any combination of (a), (b) and (c).

15. The composition of claim 14, wherein:
(a) the Tₘₐₓ is selected from the group consisting of not greater than about 90%, not greater than about 80%, not greater than about 70%, not greater than about 60%, not greater than about 50%, not greater than about 30%, not grater than about 25%, not greater than about 20%, not greater than about 15%, not greater than about 10%, and not greater than about 5% of the Tₘₐₓ exhibited by a non-nanoparticulate composition of the same ebastine, administered at the same dosage;
(b) the Cₘₐₓ is selected from the group consisting of at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by a non-nanoparticulate composition of the same ebastine, administered at the same dosage;
(c) the AUC is selected from the group consisting of at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 650%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate formulation of the same ebastine, administered at the same dosage; or
(d) any combination of (a), (b), and (c).

16. The composition of any one of claims 1 to 15 which does not produce significantly different absorption levels when administered under fed as compared to fasting conditions.

17. The composition of claim 16, wherein the difference in absorption of the active agent composition of the invention, when administered in the fed versus the fasted state, is selected from the group consisting of less than about 100%, less than about 90%, less than about 80%, less than about 70%, less than about 60%, less than about 50%, less than about 40%, less than about 30%, less than about 25%, less than about 20%, less than about 15%, less than about 10%, less than about 5%, and less than about 3%.

18. The composition of any one of claims 1 to 17, wherein administration of the composition to a human in a fasted state is bioequivalent to administration of the composition to a subject in a fed state.

19. The composition of claim 18, Wherein "bioequivalency" is established by:
(a) a 90% Confidence Interval of between 0.80 and 1.25 for both Cₘₐₓ and AUC; or
(b) a 90% Confidence Interval of between 0.80 and 1.25 for AUC and a 90% Confidence Interval of between 0.70 to 1.43 for Cₘₐₓ.

20. Use of a composition according to any one of claims 1 to 19 for the manufacture of a medicament.

21. The use of claims 20, wherein the medicament is useful in treating seasonal and perennial allergic rhinitis, idiopathic chronic urticaria, or a related disease.

22. A method of making a nanoparticulate ebastine, or a salt thereof, composition comprising contacting particles of an ebastine with at least one surface stabilizer for a time and under conditions sufficient to provide a nanoparticulate ebastine composition heaving an effective average particle size of less than about 2000 nm.

23. The method of claim 22, wherein the contacting comprises milling, wet milling, homogenizing, precipitation, freezing, supercritical fluid particle generation techniques, or emulsion techniques.

## Patentansprüche

1. Stabile, nanopartikuläre Zusammensetzung von Ebastin oder einem Salz davon, umfassend:
(a) Partikel von Ebastin oder einem Salz davon mit einer effektiven mittleren Partikelgröße von weniger als etwa 2000 nm; und
(b) mindestens einen Oberflächenstabilisator.

2. Zusammensetzung nach Anspruch 1, wobei das Ebastin in einer kristallinen Phase, einer amorphen Phase, einer semikristallinen Phase, einer semiamorphen Phase, oder Mischungen davon ist.

3. Zusammensetzung nach Anspruch 1 oder Anspruch 2, wobei die effektive mittlere Partikelgröße der Partikel von Ebastin oder einem Salz davon ausgewählt ist aus der Gruppe bestehend aus weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm.

4. Zusammensetzung nach einem beliebigen der Ansprüche 1 bis 3, wobei die nanopartikuläre Ebastinzusammensetzung verbesserte Bioverfügbarkeit verglichen mit herkömmlichen Ebastintabletten hat.

5. Zusammensetzung nach einem beliebigen der Ansprüche 1 bis 4, wobei die Zusammensetzung formuliert ist:
(a) zur Verabreichung, ausgewählt aus der Gruppe bestehend aus oraler, pulmonaler, intravenöser, rektaler, ophthalmischer, Kolon-, parenteraler, intracisternaler, intravaginaler, intraperitonealer, lokaler, buccaler, nasaler und topischer Verabreichung;
(b) in eine Dosierungsform, ausgewählt aus der Gruppe bestehend aus Flüssigdispersionen, Gelen, Aerosolen, Salben, Cremes, Tabletten, Portionsbeuteln und Kapseln;
(c) in eine Dosierungsform, ausgewählt aus der Gruppe bestehend aus lyophilisierten Formulierungen, schnell schmelzenden Formulierungen, Formulierungen mit kontrollierter Freisetzung, Formulierungen mit verzögerter Freisetzung, Formulierungen mit verlängerter Freisetzung, Formulierungen mit pulsierender Freisetzung und Formulierungen mit gemischt unmittelbarer Freisetzung und kontrollierter Freisetzung; oder
(d) eine beliebige Kombination von (a), (b) und (c).

6. Zusammensetzung nach einem beliebigen der Ansprüche 1 bis 5, wobei die Zusammensetzung weiterhin einen oder mehrere pharmazeutisch verträgliche Hilfsstoffe, Träger oder eine Kombination davon umfasst.

7. Zusammensetzung nach einem beliebigen der Ansprüche 1 bis 6, wobei:
(a) die Menge an Ebastin ausgewählt ist aus der Gruppe bestehend aus von etwa 99,5 Gew.-% bis etwa 0,001 Gew.-%, von etwa 95 Gew.-% bis etwa 0,1 Gew.-% und von etwa 90 Gew.-% bis etwa 0,5 Gew.-%, basierend auf dem kombinierten Gesamtgewicht von Ebastin und mindestens eines Oberflächenstabilisators, nicht einschließend andere Hilfsstoffe;
(b) mindestens ein Oberflächenstabilisator in einer Menge vorliegt, ausgewählt aus der Gruppe bestehend aus 0,01 Gew.-% bis etwa 99,5 Gew.-%, von etwa 0,1 Gew.-% bis etwa 95 Gew.-%, von etwa 0,5 Gew.-% bis etwa 90 Gew.-%, von etwa 5,0 Gew.-% bis etwa 99,9 Gew.-% und von etwa 10 Gew.-% bis etwa 99,5 Gew.-%, basierend auf dem kombinierten Gesamttrockengewicht an Ebastin und mindestens eines Oberflächenstabilisators, nicht einschließend andere Hilfsstoffe; oder
(c) eine Kombination von (a) und (b).

8. Zusammensetzung nach einem beliebigen der Ansprüche 1 bis 7, umfassend mindestens einen primären Oberflächenstabilisator und mindestens einen sekundären Oberflächenstabilisator.

9. Zusammensetzung nach einem beliebigen der Ansprüche 1 bis 8, wobei mindestens ein Oberflächenstabilisator ausgewählt ist aus der Gruppe bestehend aus einem nicht-ionischen Oberflächenstabilisator, einem ionischen Oberflächenstabilisator, einem anionischen Oberflächenstabilisator, einem kationischen Oberflächenstabilisator und einem zwitterionischen Oberflächenstabilisator.

10. Zusammensetzung nach einem beliebigen der Ansprüche 1 bis 9, wobei mindestens ein Oberflächenstabilisator ausgewählt ist aus der Gruppe bestehend aus Cetylpyridiniumchlorid, Gelatine, Casein, Phosphatiden, Dextran, Glycerin, Akaziengummi, Cholesterin, Tragacanth, Stearinsäure, Benzalkoniumchlorid, Calciumstearat, Glycerinmonostearat, Cetostearylalkohol, Cetomacrogol emulgierendes Wachs, Sorbitanestem, Polyoxyethylenalkylethern, Polyoxyethylenkastorölderivaten, Polyoxyethylensorbitanfettsäureestem, Polyethylenglykolen, Dodecyltrimethylammoniumbromid, Polyoxyethylenstearaten, kolloidalem Silicondioxid, Phosphaten, Natriumdodecylsulfat, Carboxymethylcellulose-Calcium, Hydroxypropylcellulosen, Hypromellose, Carboxymethylcellulose-Natrium, Methylcellulose, Hydroxyethylcellulose, Hypromellosephthalat, nicht-kristalliner Cellulose, Magnesiumaluminiumsilikat, Triethanolamin, Polyvinylalkohol, Polyvinylpyrrolidon, 4-(1,1,3,3-Tetramethylbutyl)-phenolpolymer mit Ethylenoxid und Formaldehyd, Poloxameren; Poloxaminen, einem geladenen Phospholipid, Dioctylsulfosuccinat, Dialkylesters der Natriumsulfobemsteinsäure, Natriumlaurylsulfat, Alkylarylpolyethersulfonaten, Mischungen von Saccharosestearat und Saccharosedistearat, C₁₈H₃₇CH₂C(O)N(CH₃)-CH₂(CHOH)₄(CH₂OH)₂, p-Isononylphenoxypoly-(Glycidol), Decanoyl-N-methylglucamid; n-Decyl-β-D-glucopyranosid; N-Decyl-β-D-maltopyranosid; N-Dodecyl-β-D-Glucopyranosid; N-Dodecyl-β-D-maltosid; Heptanoyl-N-methylglucamid; n-Heptyl-β-D-glucopyranosid; n-Heptyl-β-D-thioglucosid; n-Hexyl-β-D-glucopyranosid; Nonanoyl-N-methylglucamid; n-Nonyl-β-D-glucopyranosid; Octanoyl-N-methylglucamid; n-Octyl-β-D-glucopyranosid; Octyl-β-D-thioglucopyranosid; Lysozym, PEG-Phospholipid, PEG-Cholesterin, PEG-Cholesterinderivat, PEG-Vitamin A, PEG-Vitamin E, Zufalls-Copolymeren von Vinylacetat und Vinylpyrrolidon, einem kationischen Polymer, einem kationischen Biopolymer, einem kationischen Polysacharid, einem kationischen Celluloseerzeugnis, einem kationischen Alginat, einer kationischen nichtpolymeren Verbindung, einem kationischen Phospholipid, kationischen Lipiden, Polymethylmetacrylattrimethylammoniumbromid, Sulfoniumverbindungen, Polyvinylpyrrolidon-2-dimethylaminoethylmethacrylatdimethylsulfat, Hexadecyltrimethylammoniumbromid, Phosphoniumverbindungen, quaternären Ammoniumverbindungen, Benzyl-di-(2-chlorethyl)ethylammoniumbromid, Kokosnuss-Trimethylammoniumchlorid, Kokosnuss-Trimethylammoniumbromid, Kokosnuss-Methyldihydroxyethylammoniumchlorid, Kokosnuss-Methyldihydroxyethylammoniumbromid, Decyltriethylammoniumchlorid, Decyldimethylhydroxyethylammoniumchlorid, Decyldimethylhydroxyethylammoniumchloridbromid, C₁₂₋₁₅-Dimethylhydroxyethylammoniumchlorid, C₁₂₋₁₅-Dimethylhydroxyethylammoniumchloridbromid, Kokosnuss-Dimethylhydroxyethylammoniumchlorid, Kokosnuss-Dimethylhydroxyethylammoniumbromid, Myristyltrimethylammoniummethylsulfat, Lauryldimethylbenzylammoniumchlorid, Lauryldimethylbenzylammoniumbromid, Lauryldimethyl(ethenoxy)₄-ammoniumchlorid, Lauryldimethyl(ethenoxy)₄-ammoniumbromid, N-Alkyl(C₁₂₋₁₈)dimethylbenzylammoniumchlorid, N-Alkyl(C₁₄₋₁₈) dimethylbenzylammoniumchlorid, N-Tetradecyldimethylbenzylammoniumchloridmonohydrat, Dimethyldidecylammoniumchlorid, N-Alkyl- und (C₁₂₋₁₄)dimethyl-1-naphthylmethylammoniumchlorid, Trimethylammoniumhalid, Alkyl-Trimethylammoniumsalzen, Dialkyl-Dimethylammoniumsalzen, Lauryltrimethylammoniumchlorid, ethoxyliertem Alkylamidoalkyldialkylammoniumsalz, einem ethoxylierten Trialkylammoniumsalz, Dialkylbenzoldialkylammoniumchlorid, N-Didecyldimethylammoniumchlorid, N-Tetradecyldimethylbenzylammoniumchloridmonohydrat, N-Alkyl(C₁₂₋₁₄)dimethyl-1-naphthylmethylammoniumchlorid, Dodecyldimethylbenzylammoniumchlorid, Dialkylbenzolalkylammoniumchlorid, Lauryltrimethylammoniumchlorid, Alkylbenzylmethylammoniumchlorid, Alkylbenzyldimethylammoniumbromid, C₁₂-Trimethylammoniumbromiden, C₁₅-Trimethylammoniumbromiden, C₁₇-Trimethylammoniumbromiden, Dodecylbenzyltriethylammoniumchlorid, poly-Diallyldimethylammoniumchlorid (DADMAC), Dimethylammoniumchloriden, Alkyldimethylammoniumhalogeniden, Tricetylmethylammoniumchlorid, Decyltrimethylammoniumbromid, Dodecyltriethylammoniumbromid, Tetradecyltrimethylammoniumbromid, Methyltrioctylammoniumchlorid, POLYQUAT 10™, Tetrabutylammoniumbromid, Benzyltrimethylammoniumbromid, Cholinestern, Benzalkoniumchlorid, Stearalkoniumchloridverbindungen, Cetylpyridiniumbromid, Halidsalzen von quatemisierten Polyoxyethylalkylaminen, MIRAPOL™, ALKAQUAT™, Alkylpyridiniumsalzen; Aminen, Aminsalzen, Aminoxiden, Imidazoliniumsalzen, protonierten quaternären Acrylamiden, methylierten quaternären Polymeren und kationischem Guar.

11. Zusammensetzung nach einem beliebigen der Ansprüche 1 bis 10, zusätzlich umfassend einen oder mehrere, für die Behandlung von saisonaler und perennialer allergischer Rhinitis und verwandten Erkrankungen nützliche(n) Wirkstoff(e).

12. Zusammensetzung nach einem beliebigen der Ansprüche 1 bis 11, wobei:
(a) bei Verabreichung an einen Säuger die Partikel von Ebastin oder einem Salz davon so redispergieren, dass die Partikel eine effektive mittlere Partikelgröße haben, ausgewählt aus der Gruppe bestehend aus weniger als etwa 2 Mikron, weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm;
(b) die Zusammensetzung in einem biorelevanten Medium so redispergiert, dass die Ebastin-Partikel eine effektive mittlere Partikelgröße haben, ausgewählt aus der Gruppe bestehend aus weniger als etwa 2 Mikron, weniger als etwa 1900 nm, weniger als etwa 1800 nm, weniger als etwa 1700 nm, weniger als etwa 1600 nm, weniger als etwa 1500 nm, weniger als etwa 1400 nm, weniger als etwa 1300 nm, weniger als etwa 1200 nm, weniger als etwa 1100 nm, weniger als etwa 1000 nm, weniger als etwa 900 nm, weniger als etwa 800 nm, weniger als etwa 700 nm, weniger als etwa 600 nm, weniger als etwa 500 nm, weniger als etwa 400 nm, weniger als etwa 300 nm, weniger als etwa 250 nm, weniger als etwa 200 nm, weniger als etwa 100 nm, weniger als etwa 75 nm und weniger als etwa 50 nm; oder
(c) eine Kombination von (a) und (b).

13. Zusammensetzung nach Anspruch 12, wobei das biorelevante Medium ausgewählt ist aus der Gruppe bestehend aus Wasser, wässrigen Elektrolytlösungen, wässrigen Lösungen eines Salzes, wässrigen Lösungen einer Säure, wässrigen Lösungen einer Base und Kombinationen davon.

14. Zusammensetzung nach einem beliebigen der Ansprüche 1 bis 13, wobei:
(a) die Tₘₐₓ der nanopartiulären Ebastin-Zusammensetzung, wenn in dem Plasma eines Säugersubjekts nach Verabreichung getestet, geringer ist als die Tₘₐₓ für eine nicht nanopartikuläre Zusammensetzung des gleichen Ebastins, verabreicht in der gleichen Dosierung;
(b) die Cₘₐₓ der nanopartikulären Ebastin-Zusammensetzung, wenn in dem Plasma eines Säugersubjekts nach Verabreichung getestet, größer ist als die Cₘₐₓ für eine nicht nanopartikuläre Zusammensetzung des gleichen Ebastin, verabreicht bei der gleichen Dosierung;
(c) die AUC der nanopartikulären Ebastin-Zusammensetzung, wenn in dem Plasma eines Säugersubjekts nach Verabreichung getestet, größer ist als die AUC für eine nicht nanopartikuläre Zusammensetzung des gleichen Ebastin, verabreicht bei der gleichen Dosierung; oder
(d) eine beliebige Kombination von (a), (b) und (c).

15. Zusammensetzung nach Anspruch 14, wobei:
(a) die T_{maX} ausgewählt ist aus der Gruppe bestehend aus nicht größer als etwa 90 %, nicht größer als etwa 80 %, nicht größer als etwa 70 %, nicht größer als etwa 60 %, nicht größer als etwa 50 %, nicht größer als etwa 30 %, nicht größer als etwa 25 %, nicht größer als etwa 20 %, nicht größer als etwa 15 %, nicht größer als etwa 10 % und nicht größer als etwa 5 % der Tₘₐₓ, die von einer nicht nanopartikulären Zusammensetzung des gleichen Ebastins, verabreicht bei der gleichen Dosierung, aufgewiesen wird;
(b) die Cₘₐₓ ausgewählt ist aus der Gruppe bestehend aus mindestens etwa 50 %, mindestens etwa 100 %, mindestens etwa 200 %, mindestens etwa 300 %, mindestens etwa 400 %, mindestens etwa 500 %, mindestens etwa 600 %, mindestens etwa 700 %, mindestens etwa 800 %, mindestens etwa 900 %, mindestens etwa 1000 %, mindestens etwa 1100 %, mindestens etwa 1200 %, mindestens etwa 1300 %, mindestens etwa 1400 %, mindestens etwa 1500 %, mindestens etwa 1600 %, mindestens etwa 1700 %, mindestens etwa 1800 %, oder mindestens etwa 1900 % größer als die Cₘₐₓ, die von einer nicht nanopartikulären Zusammensetzung des gleichen Ebastins, verabreicht bei der gleichen Dosierung, aufgewiesen wird;
(c) die AUC ausgewählt ist aus der Gruppe bestehend aus mindestens etwa 25 %, mindestens etwa 50 %, mindestens etwa 75 %, mindestens etwa 100 %, mindestens etwa 125 %, mindestens etwa 150 %, mindestens etwa 175 %, mindestens etwa 200 %, mindestens etwa 225 %, mindestens etwa 250 %, mindestens etwa 275 %, mindestens etwa 300 %, mindestens etwa 350 %, mindestens etwa 400 %, mindestens etwa 450 %, mindestens etwa 500 %, mindestens etwa 550 %, mindestens etwa 600 %, mindestens etwa 650 %, mindestens etwa 700 %, mindestens etwa 750 %, mindestens etwa 800 %, mindestens etwa 850 %, mindestens etwa 900 %, mindestens etwa 950 %, mindestens etwa 1000 %, mindestens etwa 1050 %, mindestens etwa 1100 %, oder mindestens etwa 1150 %, oder mindestens etwa 1200 % größer als die AUC, die von der nicht nanopartikulären Formulierung des gleichen Ebastins, verabreicht bei der gleichen Dosierung, aufgewiesen wird; oder
(d) eine beliebige Kombination von (a), (b) und (c).

16. Zusammensetzung nach einem beliebigen der Ansprüche 1 bis 15, welche signifikant unterschiedliche Absorptionsspiegel nicht erzeugt, wenn sie unter gesättigten im Vergleich zu fastenden Bedingungen verabreicht wird.

17. Zusammensetzung nach Anspruch 16, wobei der Unterschied in der Absorption der Wirkstoffzusammensetzung der Erfindung, wenn sie in dem gesättigten, im Vergleich zu dem fastenden Zustand verabreicht wird, ausgewählt ist aus der Gruppe ist bestehend aus weniger als etwa 100 %, weniger als etwa 90 %, weniger als etwa 80 %, weniger als etwa 70 %, weniger als etwa 60 %, weniger als etwa 50 %, weniger als etwa 40 %, weniger als etwa 30 %, weniger als etwa 25 %, weniger als etwa 20 %, weniger als etwa 15 %, weniger als etwa 10 %, weniger als etwa 5 % und weniger als etwa 3 %.

18. Zusammensetzung nach einem beliebigen der Ansprüche 1 bis 17, wobei die Verabreichung der Zusammensetzung an einen Menschen in einem fastenden Zustand bioäquivalent zu einer Verabreichung der Zusammensetzung an ein Subjekt in einem gesättigten Zustand ist.

19. Zusammensetzung nach Anspruch 18, wobei "Bioäquivalenz" hergestellt ist durch:
(a) ein 90 % Konfidenzintervall von zwischen 0,80 und 1,25 für sowohl Cₘₐₓ als auch AUC; oder
(b) ein 90 % Konfidenzintervall von zwischen 0,80 und 1,25 für AUC und ein 90 % Konfidenzintervall von zwischen 0,70 bis 1,43 für Cₘₐₓ.

20. Verwendung einer Zusammensetzung gemäß einem beliebigen der Ansprüche 1 bis 19 für die Herstellung eines Medikaments.

21. Verwendung nach Anspruch 20, wobei das Medikament nützlich zum Behandeln saisonaler und perennialer allergischer Rhinitis, idiopathischer chronischer Urticaria oder einer verwandten Erkrankung ist.

22. Verfahren zum Herstellen einer nanopartikulären Zusammensetzung von Ebastin oder einem Salz davon, umfassend das Inkontaktbringen von Partikeln eines Ebastins mit mindestens einem Oberflächenstabilisator für eine Zeit und unter Bedingungen, die ausreichend sind, um eine nanopartikuläre Ebastin-Zusammensetzung mit einer effektiven mittleren Partikelgröße von weniger als etwa 2000 nm bereitzustellen.

23. Verfahren nach Anspruch 22, wobei das Inkontaktbringen Mahlen, nasses Mahlen, Homogenisieren, Präzipitation, Einfrieren, superkritische Flüssigkeitspartikel-Generierungstechniken oder Emulsionstechniken umfasst.

## Revendications

1. Composition nanoparticulaire stable d'ébastine, ou d'un sel de celle-ci, comprenant :
(a) des particules d'ébastine ou d'un sel de celle-ci ayant une taille de particule moyenne effective inférieure à environ 2 000 nm ; et
(b) au moins un stabilisant de surface.

2. Composition selon la revendication 1, dans laquelle l'ébastine est dans une phase cristalline, une phase amorphe, une phase semi-cristalline, une phase semi-amorphe, ou les mélanges de celles-ci.

3. Composition selon la revendication 1 ou la revendication 2, dans laquelle la taille de particule moyenne effective des particules d'ébastine ou d'un sel de celle-ci est choisie dans le groupe constitué de inférieure à environ 1 900 nm, inférieure à environ 1 800 nm, inférieure à environ 1 700 nm, inférieure à environ 1 600 nm, inférieure à environ 1 500 nm, inférieure à environ 1 400 nm, inférieure à environ 1 300 nm, inférieure à environ 1 200 nm, inférieure à environ 1 100 nm, inférieure à environ 1 000 nm, inférieure à environ 900 nm, inférieure à environ 800 nm, inférieure à environ 700 nm, inférieure à environ 600 nm, inférieure à environ 500 nm, inférieure à environ 400 nm, inférieure à environ 300 nm, inférieure à environ 250 nm, inférieure à environ 200 nm, inférieure à environ 100 nm, inférieure à environ 75 nm, et inférieure à environ 50 nm.

4. Composition selon l'une quelconque des revendications 1 à 3, dans laquelle la composition nanoparticulaire d'ébastine a une biodisponibilité améliorée par rapport à des comprimés d'ébastine classiques.

5. Composition selon l'une quelconque des revendications 1 à 4, dans laquelle la composition est formulée :
(a) pour une administration choisie dans le groupe constitué d'une administration par voie orale, pulmonaire, intraveineuse, rectale, ophtalmique, colique, parentérale, intracisternale, intravaginale, intrapéritonéale, locale, buccale, nasale, et topique ;
(b) en une forme posologique choisie dans le groupe constitué des dispersions liquides, des gels, des aérosols, des pommades, des crèmes, des comprimés, des sachets et des capsules ;
(c) en une forme posologique choisie dans le groupe constitué des formulations lyophilisées, des formulations à fusion rapide, des formulations à libération contrôlée, des formulations à libération retardée, des formulations à libération prolongée, des formulations à libération intermittente, et des formulations mixtes à libération immédiate et libération contrôlée ; ou
(d) n'importe quelle combinaison de (a), (b), et (c).

6. Composition selon l'une quelconque des revendications 1 à 5, dans laquelle la composition comprend en outre un ou plusieurs excipients, supports pharmaceutiquement acceptables, ou une combinaison de ceux-ci.

7. Composition selon l'une quelconque des revendications 1 à 6, dans laquelle :
(a) la quantité d'ébastine est choisie dans le groupe constitué d'environ 99,5 % à environ 0,001 %, d'environ 95 % à environ 0,1 %, et d'environ 90 % à environ 0,5 %, en poids, sur la base du poids total combiné d'ébastine et d'au moins un stabilisant de surface, sans inclure les autres excipients ;
(b) au moins un stabilisant de surface est présent dans une quantité choisie dans le groupe constitué de 0,01 % à environ 99,5 % en poids, d'environ 0,1 % à environ 95 % en poids, d'environ 0,5 % à environ 90 % en poids, d'environ 5,0 % à environ 99,9 % en poids, et d'environ 10 % à environ 99,5 % en poids, sur la base du poids sec total combiné d'ébastine et d'au moins un stabilisant de surface, sans inclure les autres excipients ; ou
(c) une combinaison de (a) et (b).

8. Composition selon l'une quelconque des revendications 1 à 7, comprenant au moins un stabilisant de surface primaire et au moins un stabilisant de surface secondaire.

9. Composition selon l'une quelconque des revendications 1 à 8, dans laquelle au moins un stabilisant de surface est choisi dans le groupe constitué d'un stabilisant de surface non ionique, un stabilisant de surface ionique, un stabilisant de surface anionique, un stabilisant de surface cationique, et un stabilisant de surface zwitterionique.

10. Composition selon l'une quelconque des revendications 1 à 9, dans laquelle au moins un stabilisant de surface est choisi dans le groupe constitué du chlorure de cétyl pyridinium, de la gélatine, de la caséine, des phosphatides, du dextran, du glycérol, de la gomme acacia, du cholestérol, de l'adragante, de l'acide stéarique, du chlorure de benzalkonium, du stéarate de calcium, du monostéarate de glycérol, de l'alcool cétostéarylique, d'une cire émulsionnante cétomacrogol, des esters de sorbitan, des éthers d'alkyle polyoxyéthylénés, des dérivés d'huile de ricin polyoxyéthylénés, des esters d'acide gras et de sorbitan polyoxyéthylénés, des polyéthylène glycols, du bromure de dodécyle triméthyl ammonium, des stéarates polyoxyéthylénés, du dioxyde de silicium colloïdal, des phosphates, du dodécylsulfate de sodium, de la carboxyméthylcellulose calcique, des hydroxypropylcelluloses, de l'hypromellose, de la carboxyméthylcellulose sodique, de la méthylcellulose, de l'hydroxyéthylcellulose, du phtalate d'hypromellose, de la cellulose non cristalline, du silicate de magnésium et d'aluminium, de la triéthanolamine, du poly(alcool de vinyle), de la polyvinylpyrrolidone, d'un polymère de 4-(1,1,3,3-tétraméthylbutyl)-phénol avec de l'oxyde d'éthylène et du formaldéhyde, des poloxamères ; des poloxamines, d'un phospholipide chargé, d'un dioctylsulfosuccinate, des dialkylesters d'acide sulfosuccinique sodique, du lauryl sulfate de sodium, des alkyl aryle polyéther sulfonates, des mélanges de stéarate de saccharose et de distéarate de saccharose, du C₁₈H₃₇CH₂C(O)N(CH₃)-CH₂(CHOH)₄(CH₂OH)₂, du *p-*isononylphénoxypoly-(glycidol), du décanoyl-N-méthylglucamide ; du *n*-décyl β-D-glucopyranoside du *n-*décyl β-D-maltopyranoside ; du *n*-dodécyl β-D-glucopyranoside ; du *n*-dodécyl β-D-maltoside ; de l'heptanoyl-N-méthylglucamide ; du *n*-heptyl-β-D-glucopyranoside ; du *n*-heptyl β-D-thioglucoside ; du *n-*hexyl β-D-glucopyranoside ; du nonanoyl-N-méthylglucamide ; du *n*-nonyl β-D-glucopyranoside ; de l'octanoyl-N-méthylglucamide ; du *n*-octyl-β-D-glucopyranoside ; de l'octyl β-D-thioglucopyranoside ; d'un lysozyme, d'un PEG-phospholipide, d'un PEG-cholestérol, d'un dérivé PEG-cholesterol, de la PEG-vitamine A, de la PEG-vitamine E, des copolymères aléatoires d'acétate de vinyle et de vinyle pyrrolidone, d'un polymère cationique, d'un biopolymère cationique, d'un polysaccharide cationique, d'un cellulosique cationique, d'un alginate cationique, d'un composé non polymérique cationique, d'un phospholipide cationique, des lipides cationiques, du bromure de polyméthylméthacrylate triméthylammonium, des composés sulfonium, du diméthyl sulfate de polyvinylpyrrolidone-2-diméthylaminoéthyl méthacrylate, du bromure d'hexadécyltriméthylammonium, des composés de phosphonium, des composés d'ammonium quaternaire, du bromure de benzyldi(2-chloroéthyl)éthylammonium, du chlorure de triméthylammonium de noix coco, du bromure de triméthylammonium de noix coco, du chlorure de méthyl dihydroxyéthylammonium de noix de coco, du bromure de méthyl dihydroxyéthylammonium de noix de coco, du chlorure de décyl triéthylammonium, du chlorure de décyl diméthyl hydroxyéthylammonium, du bromure chlorure de décyl diméthyl hydroxyéthylammonium, du chlorure de diméthyl hydroxyéthylammonium en C₁₂₋₁₅, du bromure chlorure de diméthyl hydroxyéthylammonium en C₁₂₋₁₅, du chlorure de diméthyl hydroxyéthylammonium de noix de coco, du bromure de diméthyl hydroxyéthylammonium de noix de coco, du méthyl sulfate de myristyl triméthylammonium, du chlorure de lauryl diméthyl benzylammonium, du bromure de lauryl diméthyl benzylammonium, du chlorure de lauryl diméthyl (éthénoxy)₄ ammonium, du bromure de lauryl diméthyl (éthénoxy)₄ ammonium, d'un chlorure de N-alkyldiméthyl en (C₁₂₋₁₈)benzylammonium, d'un chlorure de N-alkyldiméthyl en (C₁₄₋₁₈) -benzylammonium, du chlorure de N-tétradécyldiméthylbenzylammonium monohydraté, du chlorure de diméthyl didécylammonium, d'un chlorure de N-alkyle et de diméthyl en (C₁₂₋₁₄) 1-naphtylméthylammonium, d'un halogénure de triméthylammonium, des sels d'alkyltriméthylammonium, des sels de dialkyl-diméthylammonium, du chlorure de lauryl triméthylammonium, d'un sel alkylamidoalkyldialkylammonium éthoxylé, d'un sel trialkyl ammonium éthoxylé, d'un chlorure de dialkylbenzène dialkylammonium, du chlorure de N-didécyldiméthyl ammonium, du chlorure N-tétradécyldiméthylbenzylammonium monohydraté, d'un chlorure de N-alkyldiméthyl en (C₁₂-₁₄) 1-naphtylméthylammonium, du chlorure de dodécyldiméthylbenzyl ammonium, d'un chlorure de dialkyl benzènealkylammonium, du chlorure de lauryl triméthylammonium, d'un chlorure d'alkylbenzyl méthylammonium, d'un bromure d'alkyl benzyl diméthylammonium, des bromures de triméthylammonium en C₁₂, des bromures de triméthylammonium en C₁₅, des bromures de triméthylammonium en C₁₇, du chlorure de dodécylbenzyl triéthylammonium, du chlorure de polydiallyldiméthylammonium (DADMAC), des chlorures de diméthylammonium, des halogénures d'alkyldiméthylammonium, du chlorure de tricétyl méthylammonium, du bromure de décyltriméthylammonium, du bromure de dodécyltriéthylammonium, du bromure de tétradécyltriméthylammonium, du chlorure de méthyl trioctylammonium, du POLYQUAT 10^{™}, du bromure de tétrabutylammonium, du bromure de benzyl triméthylammonium, des esters de choline, du chlorure de benzalkonium, des composés de chlorure de stéaralkonium, du bromure de cétyl pyridinium, des sels halogénure de polyoxyéthylalkylamines quaternisées, du MIRAPOL^{™}, de l'ALKAQUAT^{™}, des sels d'alkyl pyridinium ; des amines, des sels d'amine, des oxydes d'amine, des sels d'imide azolinium, des acrylamides quaternaires protonés, des polymères quaternaires méthylés, et de la guar cationique.

11. Composition selon l'une quelconque des revendications 1 à 10, comprenant en plus un ou plusieurs agent(s) actif(s) utile(s) pour le traitement d'une rhinite allergique saisonnière et apériodique et des maladies apparentées.

12. Composition selon l'une quelconque des revendications 1 à 11, dans laquelle :
(a) lors de l'administration à un mammifère les particules d'ébastine ou d'un sel de celle-ci se dispersent de nouveau de sorte que les particules ont une taille moyenne de particule effective choisie dans le groupe constitué de inférieure à environ 2 microns, inférieure à environ 1 900 nm, inférieure à environ 1 800 nm, inférieure à environ 1 700 nm, inférieure à environ 1 600 nm, inférieure à environ 1 500 nm, inférieure à environ 1 400 nm, inférieure à environ 1 300 nm, inférieure à environ 1 200 nm, inférieure à environ 1 100 nm, inférieure à environ 1 000 nm, inférieure à environ 900 nm, inférieure à environ 800 nm, inférieure à environ 700 nm, inférieure à environ 600 nm, inférieure à environ 500 nm, inférieure à environ 400 nm, inférieure à environ 300 nm, inférieure à environ 250 nm, inférieure à environ 200 nm, inférieure à environ 100 nm, inférieure à environ 75 nm, et inférieure à environ 50 nm.
(b) la composition se disperse de nouveau dans un milieu bioéquivalent de sorte que les particules d'ébastine ont une taille moyenne de particule effective choisie dans le groupe constitué de inférieure à environ 2 microns, inférieure à environ 1 900 nm, inférieure à environ 1 800 nm, inférieure à environ 1 700 nm, inférieure à environ 1 600 nm, inférieure à environ 1 500 nm, inférieure à environ 1 400 nm, inférieure à environ 1 300 nm, inférieure à environ 1 200 nm, inférieure à environ 1 100 nm, inférieure à environ 1 000 nm, inférieure à environ 900 nm, inférieure à environ 800 nm, inférieure à environ 700 nm, inférieure à environ 600 nm, inférieure à environ 500 nm, inférieure à environ 400 nm, inférieure à environ 300 nm, inférieure à environ 250 nm, inférieure à environ 200 nm, inférieure à environ 100 nm, inférieure à environ 75 nm, et inférieure à environ 50 nm ; ou
(c) une combinaison de (a) et (b).

13. Composition selon la revendication 12, dans laquelle le milieu bioéquivalent est choisi dans le groupe constitué de l'eau, des solutions d'électrolyte aqueuses, des solutions aqueuse d'un sel, des solutions aqueuses d'un acide, des solutions aqueuses d'une base, et des combinaisons de celles-ci.

14. Composition selon l'une quelconque des revendications 1 à 13, dans laquelle :
(a) le Tₘₐₓ de la composition nanoparticulaire d'ébastine, lorsque testé dans le plasma d'un sujet mammifère après l'administration, est inférieur au Tₘₐₓ pour une composition non nanoparticulaire de la même ébastine, administrée à la même dose ;
(b) la Cₘₐₓ de la composition nanoparticulaire d'ébastine, lorsque testé dans le plasma d'un sujet mammifère après l'administration, est supérieure à la Cₘₐₓ d'une composition non nanoparticulaire de la même ébastine, administrée à la même dose ;
(c) l'ASC de la composition nanoparticulaire d'ébastine, lorsque testé dans le plasma d'un sujet mammifère après l'administration, est supérieure à l'ASC d'une composition non nanoparticulaire de la même ébastine, administrée à la même dose ; ou
(d) n'importe quelle combinaison de (a), (b) et (c).

15. Composition selon la revendication 14, dans laquelle :
(a) le Tₘₐₓ est choisi dans le groupe constitué de non supérieur à environ 90 %, non supérieur à environ 80 %, non supérieur à environ 70 %, non supérieur à environ 60 %, non supérieur à environ 50 %, non supérieur à environ 30 %, non supérieur à environ 25 %, non supérieur à environ 20 %, non supérieur à environ 15 %, non supérieur à environ 10 %, et non supérieur à environ 5 % du Tₘₐₓ présenté par une composition non nanoparticulaire de la même ébastine, administrée à la même dose.
(b) la Cₘₐₓ est choisie dans le groupe constitué d'au moins environ 50 %, au moins environ 100 %, au moins environ 200 %, au moins environ 300 %, au moins environ 400 %, au moins environ 500 %, au moins environ 600 %, au moins environ 700 %, au moins environ 800 %, au moins environ 900 %, au moins environ 1 000 %, au moins environ 1 100 %, au moins environ 1 200 %, au moins environ 1 300 %, au moins environ 1 400 %, au moins environ 1 500 %, au moins environ 1 600 %, au moins environ 1 700 %, au moins environ 1 800 %, ou au moins environ 1 900 % supérieure à la Cₘₐₓ présentée par une composition non nanoparticulaire de la même ébastine, administrée à la même dose ;
(c) l'ASC est choisie dans le groupe constitué d'au moins environ 25 %, au moins environ 50 %, au moins environ 75 %, au moins environ 100 %, au moins environ 125 %, au moins environ 150 %, au moins environ 175 %, au moins environ 200 %, au moins environ 225 %, au moins environ 250 %, au moins environ 275 %, au moins environ 300 %, au moins environ 350 %, au moins environ 400 %, au moins environ 450 %, au moins environ 500 %, au moins environ 550 %, au moins environ 600 %, au moins environ 650 %, au moins environ 700 %, au moins environ 750 %, au moins environ 800 %, au moins environ 850 %, au moins environ 900 %, au moins environ 950 %, au moins environ 1 000 %, au moins environ 1 050 %, au moins environ 1 100 %, au moins environ 1 150 %, ou au moins environ 1 200 % supérieure à l'ASC présentée par la formulation non particulaire de la même ébastine, administrée à la même dose ; ou
(d) n'importe quelle combinaison de (a), (b) et (c).

16. Composition selon l'une quelconque des revendications 1 à 15 qui ne produit pas des taux d'absorption significativement différents lorsqu'elle est administrée dans un état alimenté par rapport à des conditions de jeûne.

17. Composition selon la revendication 16, dans laquelle la différence d'absorption de la composition d'agent actif selon l'invention, lorsqu'elle est administrée dans l'état alimenté versus à jeun, est choisie dans le groupe constitué de inférieure à environ 100 %, inférieure à environ 90 %, inférieure à environ 80 %, inférieure à environ 70 %, inférieure à environ 60 %, inférieure à environ 50 %, inférieure à environ 40 %, inférieure à environ 30 %, inférieure à environ 25 %, inférieure à environ 20 %, inférieure à environ 15 %, inférieure à environ 10 %, inférieure à environ 5 %, et inférieure à environ 3 %.

18. Composition selon l'une quelconque des revendications 1 à 17, dans laquelle l'administration de la composition à un humain dans un état à jeun est bioéquivalente à l'administration de la composition à un sujet dans un état alimenté.

19. Composition selon la revendication 18, dans laquelle une « bioéquivalence » est établie par :
(a) un intervalle de confiance à 90 % entre 0,80 et 1,25 à la fois pour la Cₘₐₓ et l'ASC ; ou
(b) un intervalle de confiance à 90 % entre 0,80 et 1,25 pour l'ASC et un intervalle de confiance à 90 % entre 0,70 et 1,43 pour la Cₘₐₓ.

20. Utilisation d'une composition selon l'une quelconque des revendications 1 à 19 pour la fabrication d'un médicament.

21. Utilisation selon la revendication 20, dans laquelle le médicament est utile dans le traitement d'une rhinite allergique saisonnière et apériodique, d'un urticaire idiopathique chronique, ou d'une maladie apparentée.

22. Procédé de fabrication d'une composition nanoparticulaire d'ébastine, ou d'un sel de celle-ci, comprenant la mise en contact de particules d'une ébastine avec au moins un stabilisant de surface pendant une durée et dans les conditions suffisantes pour fournir une composition nanoparticulaire d'ébastine ayant une taille moyenne de particule effective inférieure à environ 2 000 nm.

23. Procédé selon la revendication 22, dans lequel la mise en contact comprend un broyage, un broyage humide, une homogénéisation, une précipitation, une congélation, des techniques de production de particule en fluide supercritique, ou des techniques d'émulsion.
